# EUROPEAN PATENT SPECIFICATION

(11) **EP 2 533 844 B1**
(45) Date of publication and mention of the grant of the patent: **13.07.2016**
(21) Application number: 10845941.3
(22) Date of filing: 08.11.2010
(51) Int. Cl.: A61M 29/00, A61F 5/00

(54) **SYSTEM FOR TREATING OBESITY AND TYPE 2 DIABETES**
SYSTEM ZUR BEHANDLUNG VON ADIPOSITAS UND TYP-2-DIABETES
SYSTÈME POUR TRAITER L'OBÉSITÉ ET LE DIABÈTE DE TYPE 2

(30) Priority: 09.02.2010 US 702422; 09.02.2010 US 702411
(43) Date of publication of application: 19.12.2012
(73) Proprietor: E2, LLC, Morris Plains, NJ 07950 (US)
(72) Inventor: PRIPLATA, Attila, Somerville, Massachusetts 02144 (US); ERRICO, Joseph, Warren New Jersey 07059 (US); RAFFLE, John, Austin Texas 78746 (US); GARDINER, Jonathan, Budd Lake New Jersey 07828 (US)
(74) Representative: Richardt Patentanwälte PartG mbB
(86) International application number: PCT/US2010/055765
(87) International publication number: WO 2011/100006

(56) References cited:
- US-A- 5 820 584
- US-A- 5 820 584
- US-A1- 2004 092 892
- US-A1- 2005 192 614
- US-A1- 2005 192 629
- US-A1- 2005 273 060
- US-A1- 2006 116 736
- US-A1- 2009 198 210
- US-A1- 2009 259 237

## Description

### BACKGROUND OF THE INVENTION

The present invention relates to the field of obesity and diabetes and more specifically to minimally invasive systems for controlling or treating obesity and/or type 2 diabetes.

Obesity is one of the leading preventable causes of death worldwide and has become a global epidemic affecting more than 400 million people. In the United States alone, approximately 300,000 obesity-linked deaths occur annually, and obesity-related co-morbidities lead to nearly $150 billion in healthcare spending. Obesity is a medical condition associated with many subsequent diseases, including type-II diabetes, cardiovascular disease, sleep apnea and certain types of cancer. These conditions often have severe adverse effects on overall health, reduce quality of life, limit productivity, lead to significant medical costs, and can ultimately lead to reduced life expectancy.

While obesity has a range of contributing causes, the vast majority of obese individuals are obese because they overeat, fail to exercise adequately, and in some cases have genetic predispositions to weight gain. The primary treatment for obesity is dieting, routine physical exercise, and in some cases pharmacologic therapy. Obesity surgery, including irreversible Roux-en-Y gastric bypass (RYGB) and Laparoscopic Adjustable Gastric Banding (LAGB), involves surgical restriction of the stomach. These interventions are typically directed at either reducing the caloric intake of the patient by triggering the satiety impulse more rapidly or physically removing the ability of the individual to ingest more than a limited amount of food, or inhibiting the ability of the individual's digestive system to extract the full caloric value of the food being eaten.

The current surgical treatments for obesity, although often effective in achieving sustainable weight loss and thus reducing associated co-morbidities, involve gross anatomical reconstruction of the digestive system, which may be irreversible. Unfortunately, as has become widely publicized in the print and broadcast media, there can be significant adverse events, complications, and/or mortality associated with the most radical of these procedures (including but not limited to RYGB). In a large number of patients, subsequent surgical procedure(s) are required to address the complication(s) from the original surgery. While use of RYGB and LAGB are approved for individuals with lower BMIs (i.e., <40), the risks associated with the procedures have limited their adoption and/or use to only the morbidly obese population (>40 BMI). Recent reports indicate that there is a need to expand the options for obesity surgery in order to provide safer alternatives for individuals who are not prepared to risk the adverse consequences of radical RYGB and LAGB surgery, but for whom a surgical intervention is wholly appropriate. In fact, many individuals who could benefit from surgical intervention before their excess weight results in serious health problems forego surgery due to the significant complications and high rates of long-term adverse events leading to poor quality of life. Thus, there is a growing need for an effective and safe alternative to obesity surgery for the obese patient population worldwide.

Diabetes mellitus type 2 or type 2 diabetes is a disorder that is characterized by high blood glucose in the context of insulin resistance and relative insulin deficiency. There are an estimated 23.6 million people in the U.S. (7.8% of the population) with diabetes with 17.9 million being diagnosed, 90% of whom are type 2. With prevalence rates doubling between 1990 and 2005, CDC has characterized the increase as an epidemic. Traditionally considered a disease of adults, type 2 diabetes is increasingly diagnosed in children in parallel to rising obesity rates due to alterations in dietary patterns as well as in life styles during childhood. Type 2 diabetes is a chronic, progressive disease that has no established cure, but does have well-established treatments which can delay or mitigate the inevitable consequences of the condition. Often, the disease is viewed as progressive since poor management of blood sugar leads to a myriad of steadily worsening complications. However, if blood sugar is properly maintained, then the disease is effectively cured - that is, patients are at no heightened risk for neuropathy, blindness, or any other high blood sugar complication. Type 2 is initially treated by adjustments in diet and exercise, and by weight loss, most especially in obese patients. The amount of weight loss which improves the clinical picture is sometimes modest (2-5 kg or 4.4-11 lb); this is almost certainly due to currently poorly understood aspects of fat tissue activity, for instance chemical signaling (especially in visceral fat tissue in and around abdominal organs).

Gastric bypass procedures typically entail surgical restriction of the size of the stomach and rerouting or bypassing a proximal portion the intestine to reduce absorption of nutrients. A study of 20-years of gastric bypass patients found that 80% of those with type 2 diabetes before surgery no longer required insulin or oral agents to maintain normal glucose levels. Weight loss also occurred rapidly in many people in the study who had had the surgery. Unfortunately, gastric bypass procedures involve irreversible reconstruction of gastrointestinal anatomy and may be associated with significant adverse events, and/or mortality. In spite of the growth in the number of surgical procedures for weight loss (greater than 250,000 annually in the US), only 1.2% of eligible patients elect to undergo these invasive surgeries each year.

Many patients who could benefit from these procedures forego surgery due to the significant complications and high rates of long-term adverse events leading to poor quality of life. The estimated 0.3-2% mortality rate along with the 19% surgical complication rate for RYGB have been major barriers for expanding the use of surgery in broader patient populations.

US patent application 20050273060 A1 describes a method and apparatus for treating obesity. The apparatus includes an artificial fistula created between gastrointestinal organs such as between the stomach and the colon. The method includes selecting an implant comprising a passageway having an internal lumen within inlet end and an outlet end. The passageway is positioned passing through a first wall of first gastrointestinal organ and a second wall of the second gastrointestinal organ with the inlet end disposed within an interior of the first gastrointestinal organ and with the outlet disposed within an interior of the second gastrointestinal organ.

US patent application 005820584A describes an elongated open-ended tube having a passageway that extends from a first end to a second end for transporting partially digested food materials from the stomach for a predetermined distance below the stomach to interrupt or reduce the intermixing of digestive fluids with the partially digested food materials. The duodenum insert is anchored within a pylorus by a pair of spaced apart first and second rings in a seated and sandwiched arrangement with the pylorus.

US Patent Publication No. 2009/0259237 to Grau et al. describes a pyloric valve for inhibiting the flow of chyme through the pyloric region of the gastrointestinal tract. The pyloric valve of Grau includes a blocking portion having a plurality of disc-shaped flanges connected in series. The blocking portion may be disposed in a contracted position wherein the plurality of disc-shaped flanges is disposed in a stacked configuration and a resting position wherein the plurality of disc-shaped flanges is disposed in a linear configuration.

In view of the foregoing, there is a need in the art for new devices and methods for controlling and treating obesity and type 2 diabetes.

### SUMMARY OF THE INVENTION

The present invention provides a device for treating obesity and/or type 2 diabetes as specified in claim 1. In one aspect of the invention, a bypass device includes a hollow bypass sleeve coupled to a pair of anchors that reside on either side of the pylorus. The hollow sleeve is designed to extend from the pyloric sphincter through at least a proximal portion of a patient's small intestine. The pair of anchors includes a gastric anchor positioned in the pyloric antrum of the stomach coupled to a duodenal anchor positioned in the proximal duodenum. The gastric anchor is movable between a collapsed configuration sized and shaped for advancement through an esophagus into pyloric antrum and an expanded configuration sized and shaped for inhibiting distal movement of the gastric anchor through the pyloric sphincter.

The duodenal anchor is coupled to the hollow sleeve and movable between a collapsed configuration sized and shaped for advancement through the esophagus, the stomach and the pyloric sphincter into the proximal duodenum and an expanded configuration sized and shaped to inhibit proximal movement of the duodenal anchor through the pyloric sphincter.

A key advantage of the present invention is that the bypass device can be placed and removed endoscopically through the patient's esophagus in a minimally invasive outpatient procedure. In addition, the bypass device expands to fit securely against tissue within the GI tract such that the position of the device is substantially maintained throughout the digestive process. Thus, the device is "self-anchoring" and does not require invasive tissue fixation within the patient's GI tract, thereby reducing collateral tissue damage and minimizing its impact on the digestive process. Also, unlike other more invasive procedures such as gastric bypass, the bypass device of the present invention does not require any permanent restructuring of the GI anatomy. Once the device is removed, the patient's GI tract should begin to function normally and in the same manner as if the device were never placed in the patient.

In a preferred embodiment, the hollow bypass sleeve is sized and shaped to extend from the distal opening of the duodenal anchor through the duodenum and into a proximal portion of the jejunum of the patient. The sleeve is positioned such that partially digested food, i.e. chyme, moving through the digestive tract passes through the interior of the sleeve. This inhibits the absorption of nutrients/calories in the upper segments of the small intestine and delays mixing of chyme with digestive enzymes such that a quantity of food ingested by the patient will have a smaller caloric value with the sleeve in place.

In addition, several recent clinical studies have demonstrated that gastric bypass surgical procedures for treating obesity, including Roux-en-Y, bilio-pancreatic diversion and duodenum exclusion, show a rapid and remarkable reduction in clinical symptoms of diabetes including normalization of glucose and insulin levels. These effects occur before any changes in obesity and suggest that the duodenum may secrete molecular signals that cause insulin resistance. Supportive data has also been demonstrated in rat models of diabetes. See, Rubino and Marescaux, Annals of Surgery, 239 No. 1, 1-11 (January 2004). Thus, the sleeve is designed to mimic the effects of bypassing the proximal portion of the small intestine seen in these surgical procedures. Specifically, it is believed that the sleeve will reduce hormonal triggers that may help to down-regulate the production of glucose, thereby resulting in a nearly immediate relief of Type-II diabetes symptoms. The stabilization or elimination of Type-II diabetes symptoms will have a beneficial impact on patient health and further increase weight loss.

In a preferred embodiment, the gastric anchor defines an exterior wall housing an interior portion and an inlet in the exterior wall for delivering a fluid to the interior portion. The gastric anchor is configured for inflation by the fluid from the collapsed configuration to the inflated configuration. The gastric anchor will preferably have a substantially annular or toroidal shape in the inflated configuration with an outer diameter sized to allow the gastric anchor to rest within the distal portion of the stomach without damaging the inner walls of the stomach while preventing distal movement of the gastric anchor through the pyloric sphincter, preferably between about 30 mm to about 70 mm. Of course, the exact diameter of the gastric anchor will depend on individual patient's anatomy. The annular shape allows for chyme to pass through a central opening in the gastric anchor from the stomach to the pylorus.

Similarly, the duodenal anchor preferably defines an exterior wall housing an interior portion and an inlet in the exterior wall for delivering a fluid to the interior such that the duodenal anchor is inflatable from the collapsed configuration to the inflated configuration. The gastric anchor also preferably has a substantially annular or toroidal shape in the inflated configuration with an outer diameter sized to allow the duodenal anchor to rest within the proximal duodenum without damaging the inner walls of the duodenum while preventing proximal movement of the duodenal anchor through the pyloric sphincter, preferably between about 20 mm to about 40 mm depending on individual patient's anatomy. Again, the central hole within the duodenal anchor allows chyme to freely pass therethrough into the hollow sleeve.

The gastric and duodenal anchors are coupled to each other by one or more flexible columns that extend through the pyloric sphincter in the operative position. The flexible columns allow both anchors to move back and forth within the stomach and duodenum, respectively, with the natural peristalsis motion of the patient. In the preferred embodiments, the gastric and duodenal anchors will periodically or continuously apply slight contact pressure to the distal portion of the pyloric antrum and the proximal portion of the duodenum, respectively. In an exemplary embodiment, the flexible columns comprise a flexible material that has sufficient tensile strength to withstand the strong peristalsis forces of the patient, such as silicone or the like.

In a preferred embodiment, the gastric and duodenal anchors both comprise substantially hollow structures defining a relatively thin exterior wall surrounding a hollow interior (i.e., balloons). The balloons each include a fluid inlet, preferably comprising a one-way valve, for delivering a fluid into the interior of the balloons to inflate the balloons into their expanded configurations after the balloons have been positioned within the patient. The exterior walls of the balloons may comprise a number of suitable biocompatible materials, such as silicone and the like. The fluid is preferably a biocompatible fluid, such as isotonic saline, that can be safely expelled into the patient's GI tract upon removal of the device.

A method for treating obesity and type 2 diabetes comprises positioning a substantially hollow sleeve within a patient's body such that the sleeve extends from the pyloric sphincter through at least a proximal portion of the duodenum of the patient. A duodenal anchor coupled to the sleeve is endoscopically advanced through an esophagus, a stomach and a pyloric sphincter into a proximal region of a duodenum of the patient and then expanded into an operative configuration that prevents proximal movement of the duodenal anchor through the pyloric sphincter. A gastric anchor coupled to the duodenal anchor is advanced through the esophagus into the pyloric antrum of the stomach and expanded into an operative configuration that prevents distal movement of the gastric anchor through the pyloric sphincter.

In a preferred embodiment, the method includes advancing the anchors and sleeve through the esophagus and into the stomach in the compact configurations under the visual guidance of an endoscope (although it will be recognized by those of skill in the art that other methods of visualizing the procedure can be employed). A guidewire may be inserted through the tube prior to the device to help guide the device to the target location in the GI tract. Once in position within the stomach, the duodenal anchor is advanced via the guidewire into the proximal region of the duodenum. The sleeve is extended through a length of the duodenum and, in some embodiments, into a portion of the jejunum.

The gastric anchor is expanded into its operative configuration by delivering fluid through a fluid tube into the interior of an expandable member of the anchor and then detaching the fluid tube from the anchor and removing it from the patient. The gastric anchor may be expanded prior to advancing the other components of the bypass device into the duodenum, after they have been advanced through the pylorus or after the sleeve has been advanced to its final position in the jejunum. In the preferred embodiment, the gastric anchor will be expanded prior to advancing these components through the pylorus to ensure that the device is not accidently released into the intestines.

At this point, the duodenal anchor is expanded into its operative position. In the preferred embodiment, fluid is delivered through a fluid tube into the interior of the flow restrictor to expand the flow restrictor. As with the anchor, the fluid tube is then detached and removed from the patient. The sleeve is then advanced through the duodenum to its final position.

Other aspects, features, advantages, etc. will become apparent to one skilled in the art when the description of the invention herein is taken in conjunction with the accompanying 15 drawings.

### BRIEF DESCRIPTION OF THE DRAWINGS

For the purposes of illustrating the various aspects of the invention, there are shown in the drawings forms that are presently preferred, it being understood, however, that the invention is not limited by or to the precise data, methodologies, arrangements and instrumentalities shown, but rather only by the claims.
FIG. 1 is a view of a portion of a normal GI tract of a human;
FIG. 2 is a perspective view of a bypass device in an operative configuration according to one embodiment of the present invention;
FIG. 3 is a cross-sectional view of a gastric anchor of the bypass device of Fig. 2;
FIG. 4 is a top view of an alternative embodiment of a gastric anchor according to the present invention;
FIG. 5 illustrates a distal end of a hollow sleeve according to the present invention;
FIG. 6 is a cross-sectional view of an alternative embodiment of a duodenal anchor according to the present invention;
FIG. 7 is a top view of the duodenal anchor of FIG. 6;
FIG. 8 is a perspective view of a dissolvable proximal capsule of a delivery system according to the present invention;
FIG. 9 is a perspective view of a dissolvable distal capsule of the delivery system according to the present invention;
FIG. 10A is a partial cross-sectional view of the bypass device and the delivery system according to the present invention;
FIG. 10B is a cross-sectional view of a portion of the bypass device with fluid tubes of the delivery system still coupled thereto;
FIG. 11 illustrates the insertion of a gastroscope and guidewire through an esophagus of a patient;
FIG. 12 illustrates the bypass device and a portion of the delivery system inserted into the stomach in the esophagus of the patient;
FIG. 13 illustrates the deployment of the duodenal anchor and the sleeve into the duodenum of the patient;
FIG. 14 illustrates the inflation of the gastric and duodenal anchors;
FIG. 15 illustrates the deployment of the sleeve into the small intestines of the patient;
FIG. 16 illustrates the bypass device in place in its operative configuration in the patient;
FIG. 17 is a partial cross-sectional view of an alternative embodiment of a valve for the duodenal and gastric anchors of the bypass device;
FIG. 18 illustrates an alternative introducer tube for the delivery system of the present invention;
FIG. 19 is a perspective view of an obesity device in an operative configuration not according to the present invention;
FIG. 20 is a close-up perspective of a proximal portion of the obesity device of FIG. 19;
FIG. 21 is a cross-sectional view of yet another alternative example of an obesity device ;
FIG. 22 is cross-sectional top view of a flow restrictor of the obesity device of FIG. 29;
FIG. 23 is a cross-sectional view of another alternative embodiment of a flow restrictor for the obesity device of the present invention;
FIG. 24 is a top view of the flow restrictor of FIG. 31;
FIG. 25 illustrates the insertion of a gastroscope and guidewire through an esophagus of a patient;
FIG. 26 illustrates the obesity device inserted into the stomach through an overtube in the esophagus of the patient;
FIG. 27 illustrates the deployment of the anchor and the sleeve of obesity device into the small intestine of the patient;
FIG. 28 illustrates the obesity device in place in its operative configuration in the patient;
FIG. 29 illustrates the deflation of the flow restrictor in a step in the removal of the obesity device;
FIG. 30 illustrates the removal of the flow restrictor from the patient; and
FIG. 31 illustrates the deflation of the anchor and removal of the remaining portions of the obesity device from the patient.

### DETAILED DESCRIPTION OF THE PREFERRED EMBODIMENTS

In the present invention, systems, devices are disclosed for treating and controlling obesity and/or type II diabetes. In particular, the systems of the present invention provide an internal bypass of a proximal portion of the small intestines to inhibit contact between chyme and the bypassed small intestinal walls while allowing natural peristalsis to occur.

Diabetic foot ulcers are one of the major complications of diabetes mellitus. Foot ulcers occur in 15% of all patients with diabetes and precede 84% of all lower leg amputations. The significant increase in mortality among diabetic patients with foot ulcers observed over the past 20 years is considered to be due to the development of macro and micro vascular complications, including failure of the wound healing process.

Wound healing is a 'make-up' phenomenon for the portion of tissue that gets destroyed in any open or closed injury to the skin. Being a natural phenomenon, wound healing is usually taken care of by the body's innate mechanism of action that works reliably most of the time. The key feature of wound healing is the stepwise repair of lost extracellular matrix (ECM) that forms the largest component of the dermal skin layer. Therefore, controlled and accurate rebuilding becomes essential to avoid under or over healing that may lead to various abnormalities. But in some cases, certain disorders or physiological insults disturb the wound healing process that otherwise proceed smoothly in an orderly manner. Diabetes mellitus is one such metabolic disorder that impedes the normal steps of wound healing process. Many histopathological studies show a prolonged inflammatory phase in diabetic wounds, which causes a delay in the formation of mature granulation tissue and a parallel reduction in wound tensile strength.

High blood sugar levels prevent white blood cells, which are important in defending the body against bacteria and also in cleaning up dead tissue and cells, from functioning normally. When these cells do not function properly, wounds take much longer to heal and become infected more frequently. Also, long-standing diabetes is associated with thickening of blood vessels, which prevents good circulation including the delivery of enough oxygen and other nutrients to body tissues.

Another consequence of high blood sugar levels is that the body has difficulty producing many important components in the healing process, such as vascular endothelial growth factors (VEGF) and nitric oxide. For example, nitric oxide is known as an important stimulator of cell proliferation, maturation and differentiation. Thus, nitric oxide increases fibroblast proliferation and thereby collagen production in wound healing. Also, L-arginine and nitric oxide are required for proper cross linking of collagen fibers, via proline, to minimize scarring and maximize the tensile strength of healed tissue. Endothelial cell specific nitric oxide synthase (EcNOS) is activated by the pulsatile flow of blood through vessels. Nitric oxide produced by EcNOS, maintains the diameter of blood vessels and proper blood flow to tissues. In addition to this, nitric oxide also regulates angiogenesis, which plays a major role in wound healing. Diabetic patients exhibit reduced ability to generate nitric oxide from L-arginine. Reasons that have been postulated in the literature include accumulation of nitric oxide synthase inhibitor due to high glucose associated kidney dysfunction and reduced production of nitric oxide synthase due to ketoacidosis observed in diabetic patients and pH dependent nature of nitric oxide synthase.

The present invention provides apparatus for treating wounds in patients, particularly chronic lower limb wounds in patients lacking the innate ability to regulate glucose (e.g., diabetic patients). In one aspect of the invention, a method for treating wounds includes positioning a flexible sleeve within the patient such that the sleeve extends through at least a portion of the duodenum to inhibit contact between chyme passing therethrough and at least a portion of an inner surface of the duodenum. The hollow sleeve is maintained within the duodenum for a sufficient period of time to reduce a blood glucose level in the patient. Preferably, the sleeve will be maintained in position for a sufficient period of time to permit the normalization of blood glucose levels in the patient (i.e., reducing such glucose levels to below about 150 mg, preferably below about 125 mg).

The sleeve creates an "internal bypass" that substantially inhibits contact between chyme and other substances entering the duodenum from the stomach of the patient and the bypassed portion of the duodenum. This reduces hormonal triggers that help to down-regulate the production of glucose, thereby resulting in the relief of certain symptoms that inhibit the body from effectively healing the wound, such as decreased peripheral vascular perfusion, neuropathy, a compromised or "inactive" immune system and a reduction in important components of healing, such as nitric oxide, vascular endothelial growth factors and the like.

In certain embodiments, the sleeve will be maintained in position for a sufficient period of time to increase the peripheral vascular perfusion in the patient. Patients lacking the innate ability to regulate glucose levels often suffer from decreased arterial perfusion of the extremities. The lack of blood flow to the extremities inhibits healing of wounds, such as foot ulcers and the like. In one embodiment of the present invention, the flexible sleeve is held in position within the duodenum until this process begins to reverse, thereby increasing peripheral vascular perfusion and allowing for accelerated healing of the wound.

In other embodiments, the sleeve will be maintained in position for a sufficient period of time to elevate an immune system response in the patient. Abnormally high glucose levels cause the body's immune system to become compromised or less active than normal. The white blood cells or leukocytes of the immune system that defend the body against infection become sluggish and unable to effectively fight infections, such as those associated with wounds. In this embodiment, the flexible sleeve is held in position within the duodenum until the activity of circulating leukocytes begins to substantially increase, which allows the patient's immune system to do its natural job of fighting the infection and accelerate healing of the wound.

In other embodiments, the flexible sleeve is maintained in position for a sufficient period of time to increase certain healing factors, such as nitric oxide and vascular endothelial growth factors (VEGF) in the patient. VEGF is an important signaling protein that stimulates the growth of new blood vessels, which can be a critical part of the angiogenesis process in wound healing. In one embodiment of the present invention, the flexible sleeve is held in position within the duodenum until a sufficient amount of VEGF are produced in the patient to accelerate healing of the wound.

In other embodiments, the flexible sleeve will be maintained in position for a sufficient period of time to halt or reverse neuropathy. Diabetic neuropathy is a common complication of patients lacking the innate ability to regulate glucose in which nerves are temporarily or permanently damaged as a result of high blood sugar levels (hyperglycemia). Neuropathy can complicate the wound healing process, particularly in the extremities. In this embodiment, the sleeve is held in position until nerves that have not been permanently damaged or destroyed can be repaired by the body to accelerate healing of the wound.

In a preferred embodiment, the flexible sleeve is sized and shaped to extend from the distal opening of the duodenal anchor through at least a portion of the duodenum (i.e., between about 10,16-30,48 cm (about 4-12 inches). In some embodiments, the sleeve may extend throughout the duodenum and into a proximal portion of the jejunum of the patient (i.e., between about 30,48-76,2 cm (30 inches), preferably about 30,48-40,64 cm (12-16 inches). The sleeve is substantially hollow and positioned such that partially digested food, i.e. chyme, moving through the digestive tract passes through the interior of the sleeve. This inhibits the absorption of nutrients/calories in the upper segments of the small intestine and delays mixing of chyme with digestive enzymes such that a quantity of food ingested by the patient will have a smaller caloric value with the sleeve in place. In addition, it is believed that the sleeve inhibits certain hormonal triggers that would otherwise occur when food passes through the duodenum and proximal jejunum; hormonal triggers that cause the body to become insulin resistant and result in type 2 diabetes.

In certain embodiments, the sleeve is removably introduced through a natural orifice in the patient into the small intestines, preferably endoscopically through the patient's esophagus, stomach and pylorus. The sleeve can be maintained in position within the duodenum in a variety of ways known to those of skill in the art (e.g., sutures, hooks, barbs, atraumatic anchoring mechanisms and the like), typically for about 2 weeks to one year, preferably between about 1 month to 6 months and more preferably between about 6-12 weeks.

In a preferred embodiment, the sleeve is maintained in position with a pair of anchor elements flexibly coupled to each other and the sleeve. In the preferred embodiment, the 3 0flexible anchors are endoscopically introduced with the sleeve and positioned on either side of the pylorus. The duodenal anchor element is preferably expanded to a size that will inhibit proximal movement of the duodenal anchor through the pyloric sphincter to ensure that the sleeve remains in the patient's intestines. Likewise, the gastric anchor element is expanded to a size that will inhibit or prevent distal movement through the pyloric sphincter to ensure that the device does not pass further into the intestines and create a blockage. In a preferred embodiment, the anchor elements are expanded by delivering a fluid into each anchor element to inflate said anchors. In other embodiments, the anchor elements may be expanded mechanically or by other suitable means.

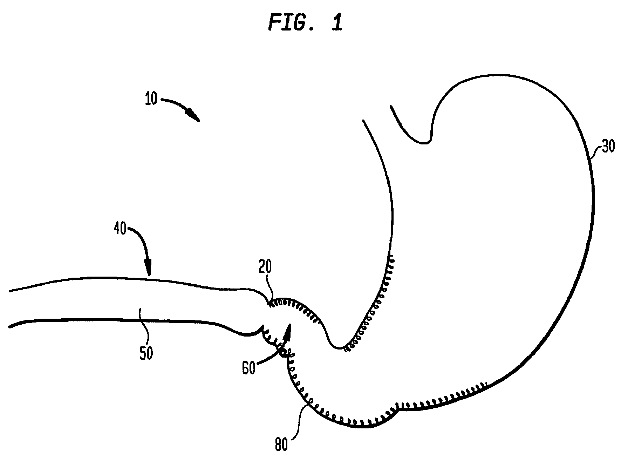

Referring to the drawings, wherein like reference numerals refer to like elements, there is shown in FIG. **1** an example of a portion of a GI tract **10** of a human body. Two smooth muscle valves, or sphincters, contain the contents of the stomach within the stomach upon ingestion. They are the esophageal sphincter (not shown), found in the cardiac region above the antrum cardiacum, and the pyloric sphincter **20,** disposed between the stomach **30** and the small intestine **40.** The pylorus **20** is the region of the stomach **30** that connects to the duodenum **50.** The pylorus **20** is divided into two parts: the pyloric antrum **60** which connects the body to the stomach and the pyloric canal which connects to the duodenum **50.** The pyloric sphincter **20** is a strong ring of smooth muscle at the end of the pyloric canal that functions to help regulate the passage of chyme from stomach **30** to the duodenum **50.**

Satiety receptors **80** are generally located all along the inside lining of stomach tissue. Partially undigested food in GI tract **10** is generally referred to as chyme. If chyme remains in the region of the stomach before flowing into small intestine **40,** satiety receptors **80** have a greater chance of being activated, which enhances the ability of an overweight or obese patient to feel satiated and suppresses the desire to eat.

Pyloric antrum **60** and duodenum **50** are innervated by the enteric nervous system and the parasympathetic nervous system (i.e., the vagus nerve). Many researchers have shown that the vagus nerve is responsible for the majority of afferent signals responsible for satiety. Thus, it is believed that increasing the afferent vagal nerve activity will result in satiety signals produced by the brain, making the patient feel more full and less inclined to eat.

A bypass device according to any of the below-described embodiments is preferably comprised of a polymeric structure that is compliant and generally flexible and bendable.

Preferably, at least a portion of the device is made of silicone. Some portions of the device may be thicker than others for enhanced strength properties and to enhance the capability of the device to resist the natural peristaltic action of GI tract **10.** Alternatively, some portions of the device may be thinner than others to allow for the material peristaltic actions of GI tract **10** to occur without the device providing a counteractive force. Preferably, if a portion of the device is bent or twisted during insertion, its polymeric structure will allow it to revert back to its resting or initial shape.

The one or more materials that comprise a bypass device according to the present invention are preferably selected for their ability to yield and flex during implantation and removal of the device. These properties also protect the patient and the tissues and organs with which the device comes into contact. The compliant nature of the bypass device allows its configuration to be manipulated during a surgical procedure, preferably in such a way that the device tends to revert to its operative configuration. The device may be made of shape memory material, such as nitinol or other known pliable polymeric materials, to allow for expansion back into its operative configuration. Any or all of the device may be coated with Teflon to provide a smooth outer surface to reduce friction between the device and the patient during implantation and removal.

The bypass device according to the present invention is structured to inhibit the rate that chyme passes through GI tract **10,** thereby enhancing the ability of chyme to activate satiety receptors **80** and effectively enhance satiety in a patient. In particular, the device is preferably structured to reduce the rate of gastric emptying such that obesity can be controlled by controlling satiety.

The bypass device of the present invention is also designed to provide periodic or continuous contact pressure on the pyloric antrum and or the proximal portion of the duodenum. This contact pressure modulates (preferably stimulates) one or more nerves within these two structures, thereby increasing their activity. In certain embodiments, this contact pressure is brought about by the design of the device; namely, the flow restrictor and anchor are coupled to each other by flexible elements or columns (discussed in detail below). These flexible columns are sized such that the anchor generally rests against the proximal portion of the duodenum and the flow restrictor generally rests against the distal portion of the pyloric antrum. The flexible columns also have enough "give" or flexibility to allow the anchor and flow restrictor to move back and forth with the peristaltic motion of the GI tract. Thus, the anchor and flow restrictor may periodically move away from the proximal portion of the duodenum and the distal portion of the pyloric antrum, respectively. However, they will generally move back in contact with these structures to provide at least periodic pressure contact on these structures to stimulate the vagus nerves therein.

The bypass device is also designed to inhibit contact between chyme passing through the duodenum and the inner walls of the duodenum. This inhibits the absorption of nutrients/calories in the upper segments of the small intestine and delays mixing of chyme with digestive enzymes such that a quantity of food ingested by the patient will have a smaller caloric value with the sleeve in place. In addition, this reduces hormonal triggers that may help to down-regulate the production of glucose, thereby resulting in a nearly immediate relief of Type-II diabetes symptoms. The stabilization or elimination of Type-II diabetes symptoms will have a beneficial impact on patient health and further increase weight loss.

Incretins are gastrointestinal hormones, produced in response to the transit of nutrients that boost insulin production. Because an excess of insulin can determine hypoglycemia (extremely low levels of blood sugar) - a life-threatening condition, it has been speculated that the body has a counter-regulatory mechanism (or "anti-incretin" mechanism), activated by the same passage of nutrients through the upper intestine. The latter mechanism would act to decrease both the secretion and the action of insulin. Thus, in healthy patients, a correct balance between incretin and anti-incretin factors maintains normal excursions of sugar levels in the bloodstream. In some individuals, however, the duodenum and jejunum may be producing too much of this anti-incretin, thereby reducing insulin secretion and blocking the action of insulin, ultimately resulting in Type 2 diabetes. Indeed, in Type 2 diabetes, cells are resistant to the action of insulin ("insulin resistance"), while the pancreas is unable to produce enough insulin to overcome the resistance. After gastrointestinal bypass procedures, the exclusion of the upper small intestine from the transit of nutrients may offset the abnormal production of anti-incretin, thereby resulting in remission of diabetes. However, it should be noted that the scientific community has not settled on a particular mechanism of action that causes patients undergoing such bypass procedures to more less "insulin resistant". Thus, the present invention is not limited to this particular mechanism of action or any particular mechanism of action.

Certain components of the bypass device according to the present invention may be discussed as being attached or connected to one another. Preferably, the device is constructed of one continuous piece and of one material, preferably silicone. However, two or more components of the device may be manufactured separately and subsequently assembled. If assembled, components may be glued together using a silicone-based glue.

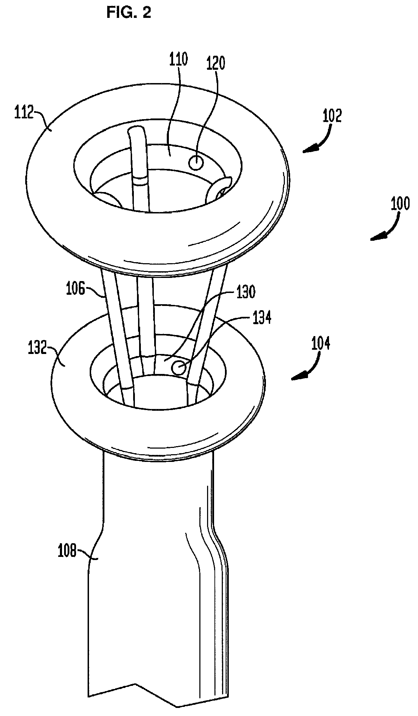

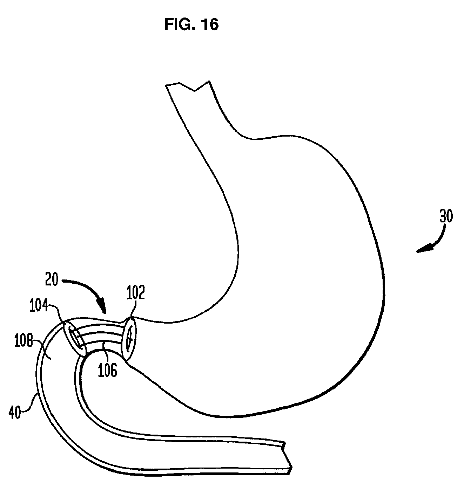

Fig. **2** illustrates one preferred embodiment of a bypass device **100** according to the present invention. As shown, device **100** includes a gastric anchor **102** coupled to a duodenal anchor **104** by a plurality of flexible silicone tethers or pyloric columns **106** and a hollow sleeve **108** coupled to the distal end of duodenal anchor **104.** Pyloric columns **106** are designed to extend through the pyloric sphincter **20** to allow both gastric anchor **102** and duodenal anchor **104** to have some limited movement back and forth within the stomach **30** and duodenum **50,** respectively, with the natural peristalsis motion of the GI tract (see FIG. **16**). Pyloric columns **106** may be cylindrical or any other type of prismic shape and are preferably designed such that the distance between the distal end of gastric anchor **102** and the proximal end of duodenal anchor **104** is about 10-60 mm, preferably about 20-40 mm, and more preferably about 30 mm, in the fully extended, but relaxed condition (i.e., non-elastically extended). Preferably, device **100** includes at least two, preferably three, pyloric columns **106** each having a diameter of about 1-5 mm which are attached to the inside surfaces of anchors **102, 104.** Alternatively, anchors **102, 104** may be coupled together with a hollow sleeve that extends through the pyloric sphincter **20.** The sleeve would preferably be sufficiently flexible to allow sphincter **20** to open and substantially close without hindrance from the sleeve.

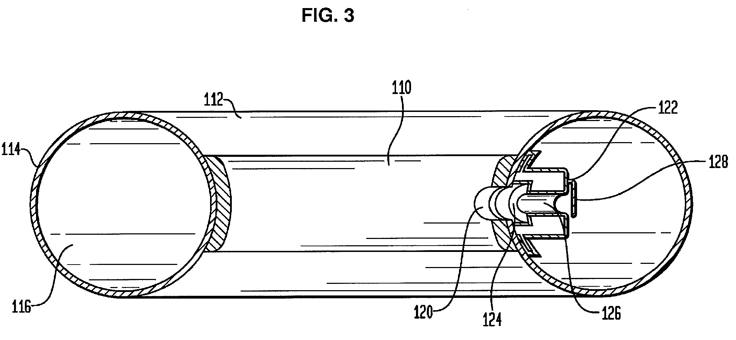

Fig. 3 illustrates a cross-sectional perspective view of an exemplary embodiment of gastric anchor **102.** As shown, gastric anchor **102** includes an internal ring 110 coupled to an annular inflatable membrane **112** having a substantially annular or toroidal shape. Inflatable membrane **112** comprises an outer wall **114** surrounding a hollow interior **116** configured for inflation via a suitable fluid. Ring **110** and membrane **112** preferably comprise a flexible biocompatible material, such as a phosphlipid resistant silicone material (e.g., a fluorosilicone copolymer). Alternatively, ring 110 may comprise a water-absorbent material, such as a hydrogel, that expands and hardens upon contact with fluid. Ring 110 provides structural support for anchor **102,** while inflatable member **112** allows anchor **102** to move between a collapsed configuration for introduction through the patient's esophagus and an expanded configuration within the stomach. Thus, ring **110** is preferably harder and less flexible than inflatable membrane **112** having a durometer of at least about 70 A, preferably greater than about 80 A, while membrane **112** preferably has a durometer between about 30-60 A, more preferably between about 40-60 A.

In an alternative embodiment, anchor **102** may simply include an inflatable member **112** without support ring **112.** In this embodiment, the inflatable member **112** would be inflated to a size and pressure sufficient to withstand peristalsis forces without compressing to a size that would allow the anchor **102** to pass through pyloric sphincter **20.**

Gastric anchor **102** further includes a fluid inlet **120** for delivery of a fluid into hollow interior **116** of membrane **112.** In the preferred embodiment, fluid inlet **120** comprises a valve **122** formed along the inner circumference of ring **110** and extending into interior **116** of membrane. As shown, valve **122** includes small chamber **124** in which a hole **126** is formed. Hole **126** extends radially from the inner surface of ring **110,** through ring **110,** into interior **116** of membrane **112.** A flexible silicone flap **128,** formed as part of the interior surface of the inflatable member **112,** is located adjacent to hole **126.** Silicone flap **128** serves to occlude hole **126** once the pressure within interior **116** of inflatable member **112** exceeds the pressure exterior to the member **112,** thereby forming a one-way valve to prevent fluid egress from membrane **112.**

In addition to, or as an alternative to, the one-way valve, a curable fluid, such as silicone, may be injected into the fluid pathway after the saline. The curable fluid will cure and harden, thereby preventing any fluid egress from the interior of inflatable member **112.** In another embodiment, the inflatable members of the anchors may comprise a material that self-seals when punctured with a very sharp small instrument such as a syringe. In this embodiment, the inflatable members of the bypass device may simply be inflated with a syringe and then self- sealed by the material to prevent fluid egress.

In the preferred embodiment, ring 110 has an inner diameter of about 10-50 mm, preferably about 20-40 mm, and more preferably about 30 mm, and a thickness of about 1-5 mm, preferably about **3** mm. Inflatable membrane **112** has an outer diameter of about 30-70 mm, preferably about 40-50, and more preferably about **45** mm, when the pressure within membrane **112** is equal to the pressure outside (the state of nominal inflation, i.e., complete inflation without elastic deformation). In the preferred embodiment, however, membrane **112** is designed such that fluid can be injected into interior **116** until it has expanded significantly beyond the initiation of elastic deformation, with a maximum exterior diameter of the implanted gastric portion preferably being between about 50 and 60 mm.

Duodenal anchor **104** preferably has a similar structure as gastric anchor **102;** including an inner ring **130** and an annular inflatable membrane **132** having a substantially annular or toroidal shape (see Fig. **2**). Inflatable membrane **132** is designed to inflate into a substantially annular shape that provides for a secure anchor against the distal opening of the pyloric sphincter. This ensures that anchor **104** will remain in place within the duodenum **50** despite the natural peristalsis forces acting against anchor **104.** Anchor **104** further includes a valve structure **134** (similar in design to gastric anchor **102b**) for delivering a fluid into an interior of membrane **132** to inflate membrane **132.** In the exemplary embodiment, ring **130** has an inner diameter of between about 10-20 mm, preferably about 15 mm, a thickness of between about 15 mm, preferably about **3** mm. Inflatable membrane **132** preferably has an outer diameter of between about 20-30 mm, preferably about 25 mm, when the pressure within membrane **132** is equal to the pressure outside (the state of nominal inflation, i.e., complete inflation without elastic deformation). Similar to the gastric component, membrane **132** is designed for inflation beyond the initiation of elastic deformation, with a maximum exterior diameter of the implanted duodenal anchor being between about 30 and 50 mm, preferably between about 35 to 40 mm.

In an alternative embodiment, gastric and duodenal anchors **102, 104** include one or more expandable components that either completely or partially replace the fluid used in the previous embodiment to inflate or expand anchors **102, 104.** In a preferred embodiment, the expandable components comprise fluid-absorbent materials designed to expand upon contact with certain fluid, such as hydrogels. Hydrogels are networks of polymer chains that are water- insoluble and hydrophilic. In this embodiment, a plurality of hydrogel components (not shown) are housed within the inflatable members of anchors **102, 104.** The hydrogel components will be introduced into the patient in the "dry" or smaller state within anchors **102, 104.** A fluid, such as saline, is delivered into the interior of the inflatable members and is absorbed within the hydrogel components (which may be of any shape, such as spheres or the like) to expand these components, thereby expanding the inflatable members.

In one aspect of this embodiment, the anchors **102, 104** include a plurality of hydrogel beads, preferably about **0.5** to **3.0** mm in diameter, designed to expand to a larger size (e.g.,16 about 4-6 mm in diameter) upon hydration with a fluid, such as saline. In this embodiment, anchors **102, 104** may or may not include inner rings **110** and they may not require one-way valves. The hydrogel balls provide additional rigidity to the anchors **102, 104.** In addition, they provide additional safety in the event that the inflatable members are punctured with a small hole as the balls will remain within inflatable members in such event (as opposed to a puncture that will allow all of the fluid to exit inflatable members in the previous embodiments). Upon removal of the device, the inflatable members will be punctured with a large enough hole to allow the hydrogel balls to exit the interiors of the inflatable members. The hydrogel balls can be allowed to pass through the patient's GI tract and excreted naturally.

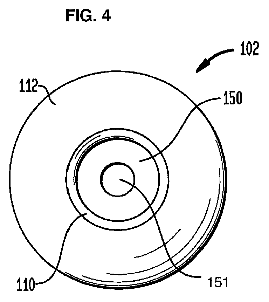

Figure **4** illustrates an alternative embodiment of gastric anchor **102.** In this embodiment, anchor **102** is substantially similar in design and construction as the anchor shown in Fig **2****,** comprising a central support ring **110** surrounded by an inflatable member **112** having an internal valve **120.** However, this anchor **102** also includes an internal restrictor plate **150** attached to support ring **110.** Plate **150** has a central hole **151** designed to allow chyme to pass therethrough. Plate **150** serves to substantially inhibit the flow of chyme from the stomach to the pyloric sphincter. It is believed that this will cause the prolongation of satiety, and result in fewer meals being eaten and/or smaller meals being ingested. The inner diameter of hole **151** will vary depending on the rate of chyme flow desired for the individual patient. For example, hole **151** may have a diameter of about 5-20 mm.

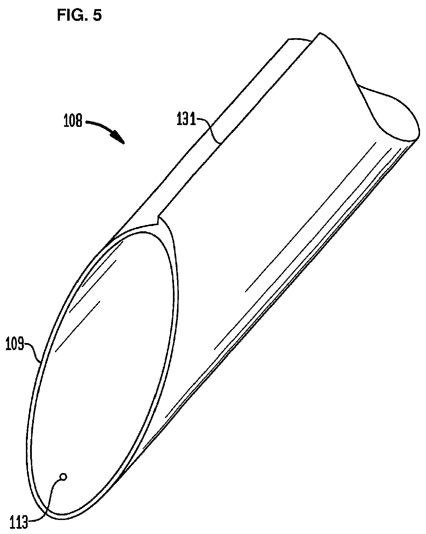

Referring to Fig. **5****,** sleeve **108** may be manufactured to any length according to a particular patient and/or surgical procedure, and in the preferred embodiment includes at its distal end a beveled tip **109.** Along its length, sleeve **108** may have one or more side holes **113** which provide further access for chyme to enter sleeve **108.** In some embodiments, a rib **131** is disposed along sleeve **108** and is preferably substantially parallel to the longitudinal axis of sleeve **108,** though rib **131** may extend only partially along sleeve **108** and may take on a curved or other type of orientation with respect to the longitudinal axis. Rib **131** may be comprised of silicone and additionally may include a radiopaque material, such as barium, so that rib **131** may be detected by a fluoroscope. Rib **131** may be provided as a separate component and later attached to sleeve **108,** or rib **131** may essentially be the overlapping seam formed during the manufacture of sleeve **108** when a flat piece of material is rolled into a tubular shape. In such a configuration, sleeve **108** may be comprised of a homogenous material attached by a radiopaque glue. Of course, as rib **131** is primarily used as an aid during implantation and/or removal of obesity device **100,** rib **131** need not necessarily be included in this or any other embodiment according to the present invention.

Sleeve **108** preferably has a diameter that will substantially correspond with the inner diameter of the patient's duodenum and a length that will allow the sleeve to extend into at least the proximal portion of the jejunum depending on the individual patient's anatomy. Thus, sleeve **108** will typically have a length of between about 20-80 cm, preferably between about 55-75 cm, a thickness of about 0.05 mm to 0.22 mm and a diameter of between about 1 to 3 cm, preferably about 2.5 cm. Preferably, the proximal portion of sleeve **108** has an inner diameter of about 1.2 cm that expands laterally in the proximal direction to 1.5 cm so that it may be sealed to the lower surface of anchor **104.** The material of the sleeve is preferably silicone, such as a polyethylene-reinforced silicone.

Sleeve **108** may include one or more markers (e.g., barium) designed for viewing the position of the sleeve within the intestines through fluoroscopy, such as rib **131** or other markers that are spaced along the length of sleeve **108.** In addition, sleeve **108** may further include components that inhibit twisting or kinking of the sleeve **108.** In one embodiment, these components include one or more stiffening elements, such as rings, coupled to either the inside or the outside of the sleeve at spaced locations along its length. These rings can, for example, be made of a slightly thicker silicone material that would resist twisting or kinking of the sleeve around the ring. In other embodiments, the stiffening elements may be in spiral shape or extending lengthwise along at least a portion of the sleeve **108.**

Sleeve **108** may also include one or more internal lumens extending along a portion of or the entire length of the sleeve. The fluid lumens comprise a proximal end configured for coupled to a fluid delivery system to allow a fluid to flow through sleeve to extend sleeve through the patient's duodenum and/or to ensure that sleeve remains patent without any twists or kinks along its length. In one embodiment, the sleeve comprises multiple (e.g., 2-5) internal lumens extending from the proximal to the distal end of the sleeve and spaced from each other around the circumference of the sleeve. In another embodiment, the sleeve comprises an internal lumen that extends in a spiral pattern down the length of the sleeve.

In yet another alternative embodiment, sleeve **108** may further include one or more fluid chamber(s) coupled to one or more of the internal lumens. The fluid chamber(s) facilitate implantation of the sleeve within the patient by allowing the physician to at least partially fill one or more of the chambers before withdrawing the scope from the duodenum (discussed in more detail below). In addition, the fluid chambers provide stiffness to the sleeve to provide more stability to the sleeve and ensure that it remains in place within the patient after implantation.

The fluid may also include a material that is observable under fluoroscopy (e.g., barium or the like) such that the location of the internal lumens and/or the fluid chambers can be viewed by a physician after implantation.

In one embodiment, the fluid chamber comprises an annular passage extending around the circumference of the sleeve at or near its distal end. This configuration provides the additional advantage that, when filled, the annular fluid chamber provides a distal anchor for the sleeve and the entire bypass device to provide additional against proximal migration of the sleeve and/or device. In another embodiment, the sleeve comprises multiple annular fluid chambers spaced along the length of the sleeve to provide additional rigidity to the sleeve, thereby ensuring that the sleeve remains patent and preventing any kinking or twisting along its length.

In an alternative implantation method, the sleeve **108** may be initially folded "accordion- style" and tucked into the interior of the anchor **104.** The distal end of sleeve **108** is initially closed, with a small silicone rubber knob (not shown) attached to the bottom of sleeve **108** (e.g., like a "sock" with a ball on the inside surface of the "toe"). Just proximal to the ball, at a region where the diameter of sleeve **108** is about 2.1 cm, is a circumferential perforation (not shown) such that the ball and the "toe portion" of the sleeve will tear away from sleeve **108** leaving an open tubular sleeve behind.

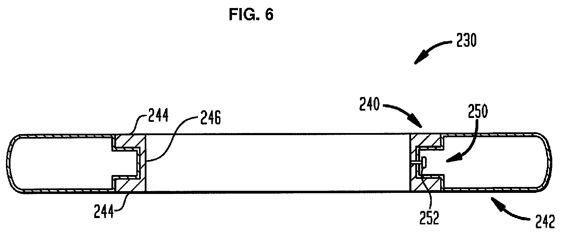

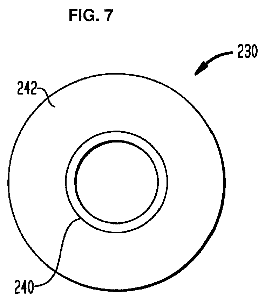

Referring now to Figs. **6** and **7****,** an alternative embodiment of a duodenal anchor **230** will now be described. As shown, anchor **230** comprises an annular support ring **240** coupled to an inflatable flexible membrane **242.** In this embodiment, support ring **240** comprises two flat ring elements **244** connected together by a semi-rigid central ring **246** that holds the flat ring elements **244** apart from each other. Flat ring elements **244** and central ring **246** preferably comprise a suitable material, such as silicone, which may be molded as one-piece or molded separated and glued together with a suitable silicone glue. Flat ring elements **244** are preferably separated from one another by a distance of about 8-12 mm. Flexible membrane **242** is sealed to the exterior circumferential surfaces of flat ring elements **244** and central ring **246.** Membrane **242** is initially tucked in a space **250** between flat ring elements **244** with sufficient additional material such that when saline is injected into the interior of membrane **242,** an inflated "inner tube" structure is created. This inner tube structure is intended to inflate to a maximum diameter of between 30-45 mm (depending on the specific duodenal anatomy of a given patient).

A one-way valve **252** is disposed between ring elements **244** at one location around the circumference thereof and directed inwardly. This valve **252** is initially coupled to a thin tube (not shown) that extends up along the exterior of the gastric anchor through which membrane **242** can be inflated. In an exemplary embodiment, a small wire (not shown) is incorporated into the seal of the inner membrane **242** to the rings to permit simple and rapid deflation of membrane **242** for removal of the device. A small ball (not shown) may also be coupled to the wire and located external to ring elements **244** and membrane **242** for grasping by an endoscopic instrument. Pulling the ball causes the wire to tear through membrane **242** and the seal, thus rupturing membrane **242** and collapsing anchor **230.**

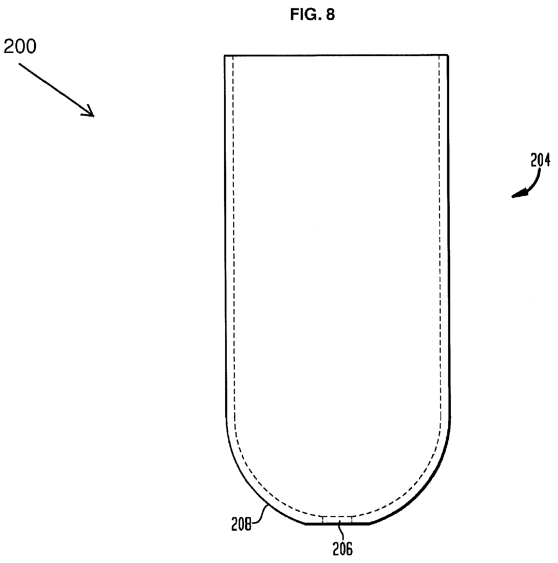

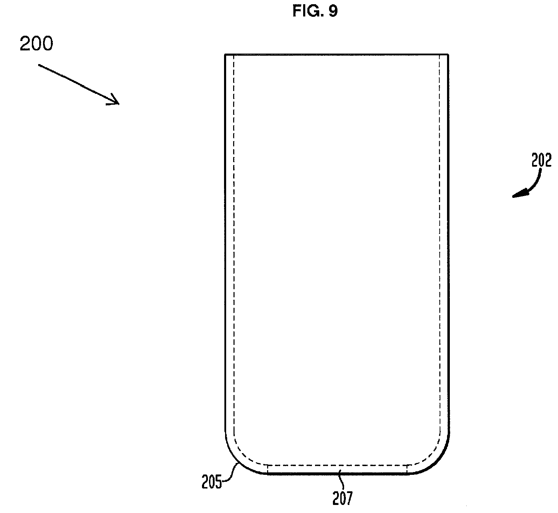

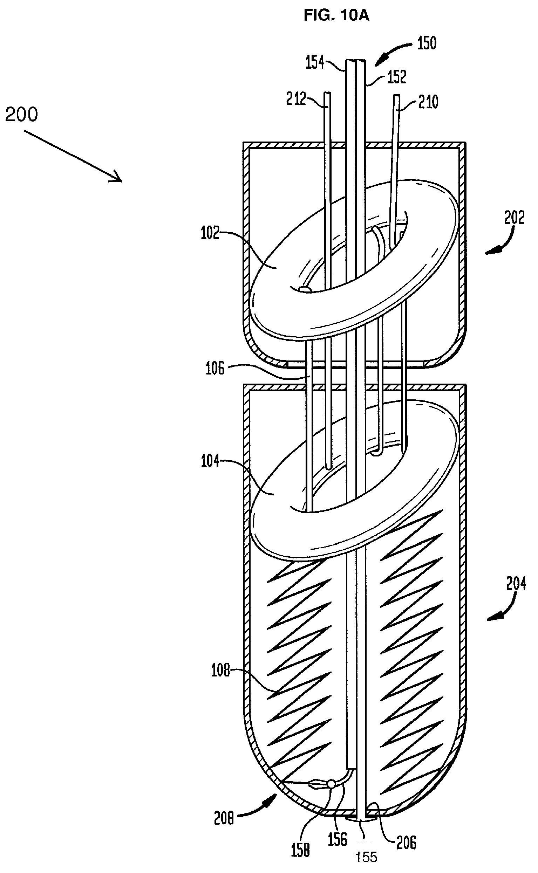

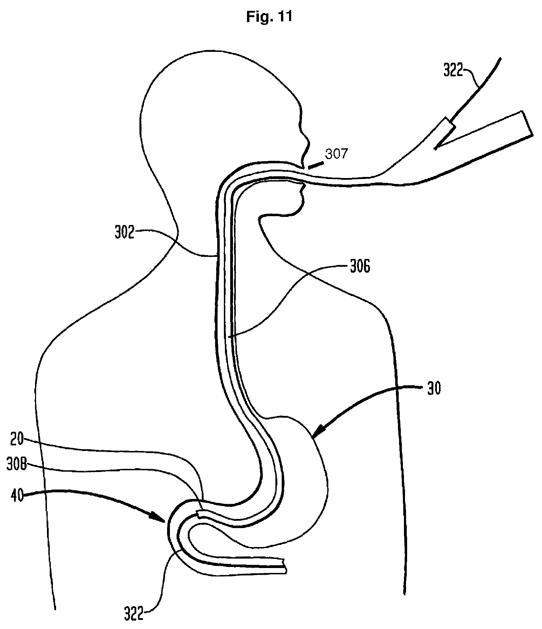

Referring now to Figs. **8-10B****,** bypass device **100** further comprises a deployment system **200** for facilitating the deployment of device **100.** As shown in Figs. **8** and **9****,** deployment system **200** includes a proximal housing or capsule **202** and a distal housing or capsule **204.** Capsules **202, 204** both comprise a biocompatible dissolvable material, such as gelatin and the like, designed to dissolve within the patient's body. As shown in Fig. **8****,** distal capsule **204** is sized and shaped to house sleeve **108** in a folded, compressed or collapsed configuration and duodenal anchor **104** in its collapsed or deflated configuration (see Fig. **10A****).** Distal capsule **204** is open at its proximal end, and has a bullet-shaped distal tip **208** with a small hole **206.** Distal tip **208** is designed to facilitate passage of the capsule **202** (and sleeve **108** and anchor **104** therein) through the pylorus and into the duodenum. Hole **206** is sized and shaped for passage of a guidewire therethrough (see FIGS. **11-15****).** Capsule **202** is preferably about 80-160 mm long (preferably about 100-140 mm long) about 10-30 mm wide (preferably about 20 mm) and is preferably constructed to survive for less than 30 minutes before dissolving in the human intestines.

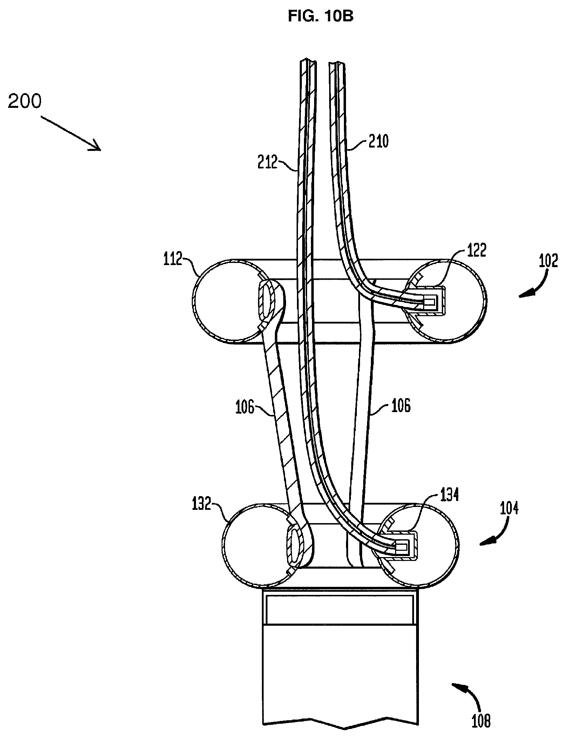

As shown in Fig. **9****,** proximal capsule **202** is sized and shaped to house gastric anchor **102** in its collapsed configuration and at least a portion of columns **106** therein (see Fig. **10A****).** Proximal capsule **202** is substantially cylindrically shaped and is primarily designed to facilitate passage of these components through the esophagus to minimize any damage to the inner walls of the esophagus. To that end, capsule **204** has a slightly curved distal end **205** with an opening **207** that is larger than distal opening **206** of distal capsule **204** to accommodate the various components of bypass device **100** therethrough, such as flexible columns **106;** delivery structure **150** and fluid tube **210** (see Fig. **10B****).** In the preferred embodiment, capsule **202** is about 30-70 mm long (preferably about 50 mm) and about 10-30 mm wide (preferably about **20** mm). Capsule **202** preferably comprises a material that will dissolve in less than 15 minutes within the human stomach, such as gelatin and the like. Alternatively, capsules **202, 204** may comprise a resorbable material or a material that can be safely excreted by the patient.

Referring now to Figs. 10A and **10B****,** deployment system **200** further includes fluid tubes **210, 212** coupled to valves **122,134** of gastric and duodenal anchors, respectively. Tubes **210, 212** each include small one-way valves (not shown), such as an umbrella valve or a duckbill valve, formed in their distal tips. Tubes **210, 212** are preferably coupled to valves **122,134** such that their own own-way valves are formed within the small chambers **124** of valves **122, 134.**

Upon inflation of the respective components, the tubes **210, 212** are then cut proximal to valves
**122, 134** such that their one-way valves provide additional protection to ensure that fluid injected into the inflatable membranes **112, 132** cannot escape through the holes.

Deployment system **200** further comprises a central tube structure **150** designed to extend through bypass device **100** (i.e., through the center of gastric and duodenal anchors **102, 104** and sleeve **108).** Central tube structure **150** has a support tube **152** with an inner lumen **(not shown)** sized for passage of a guidewire therethrough (discussed in detail below). Support tube **152** has an inner diameter of at least **2.8** mm and preferably about **3** mm. Support 20 tube **152** extends through hole **206** in distal capsule **204** and preferably has widened distal tip **155** designed to engage the outer surface of distal tip **208** of capsule **204.** This facilitates passing of guidewire into the distal opening of support tube **152.** Alternatively, support tube **152** may comprise an enlarged distal end (not shown) having a larger diameter than hole **206** that is located within capsule **204.** In this embodiment, the enlarged distal end of support tube **152** operates to push against capsule to propel capsule **204** forward as it advances through the patient's GI tract. Support tube **152** preferably comprises a material that has sufficient flexibility to easily navigate the patient's small intestines, yet sufficient rigidity to allow for a pushing force from outside of the patient's body to advance tube **152** and the rest of bypass device **100** through the patient's GI tract (discussed below). Suitable materials for support tube **152** are polypropylene and the like.

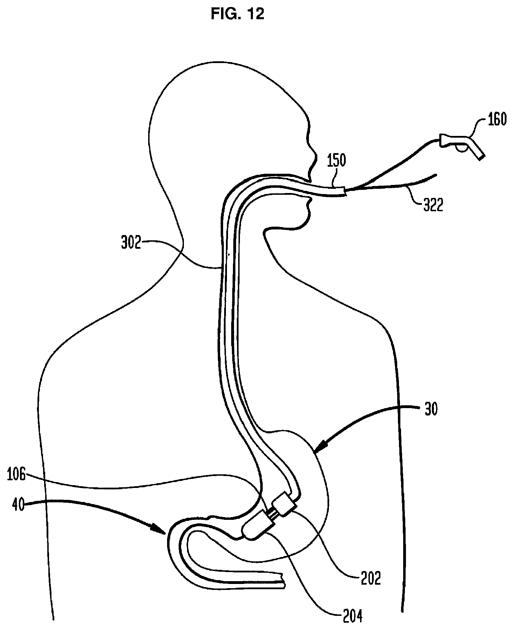

Central tube structure **150** further comprises a flexible tube **154** extending alongside support tube **150.** Flexible tube **154** houses a wire **156** coupled to a snare or a clamp **158** at its distal end. An actuator, such as a handle **160** (see Fig. **12****),** is coupled to the proximal end of wire **156** for opening and closing clamp **158.** As discussed below, flexible tube **154** and clamp **158** are designed to facilitate advancement of sleeve **108** through the patient's small intestines to its final deployment position. Note that flexible tube **154** and support tube **152** may alternatively comprise a single tubular structure with two separate lumens (one for wire **156** and one to accommodate the guidewire).

In one embodiment, sleeve **108** comprises one or more small extensions or polyps (not shown) extending from its distal tip. In this embodiment, tube **154** includes a distal tip (not shown) with a clamp or fastener designed to fasten to the polyps to facilitate advancement of sleeve **108** as discussed above. When the sleeve is in its final deployment position, an actuator on handle on the proximal end of the tube **154** allows the use to pull the clamp proximally relative to the distal tip to sever the polyps and thereby detach tube **154** from the sleeve **108.** At this point, central tube structure **150** can be removed from the patient. The polyps will then pass through the patient's GI tract in a normal manner.

In an alternative embodiment, deployment system **200** includes a thin, hollow sheath coupled to a silicone ball (not shown). The ball is detachably coupled to the distal end of sleeve **108** and the sheath extends through sleeve **108** and duodenal and gastric anchors **102, 104.** The ball has an axial hole formed therethrough aligned with the axial bore of the sheath. The axial bore of the flexible sheath has a length of about 120 mm, and an outer diameter of about 5 mm. The sheath and ball together allow the user to advance sleeve **108** through the intestines to its final deployment position.

In reference to Figs. **11-16****,** a method of implanting and removing a bypass device 100 according to the present invention will now be described. While the description of this method will be specifically directed to the embodiments illustrated in Figs. **2** and **3****,** it will be understood by those skilled in the art that this method (or similar methods) can be used to implant and remove all of the embodiments of the present invention, including embodiments or designs that may not be specifically described or illustrated herein.

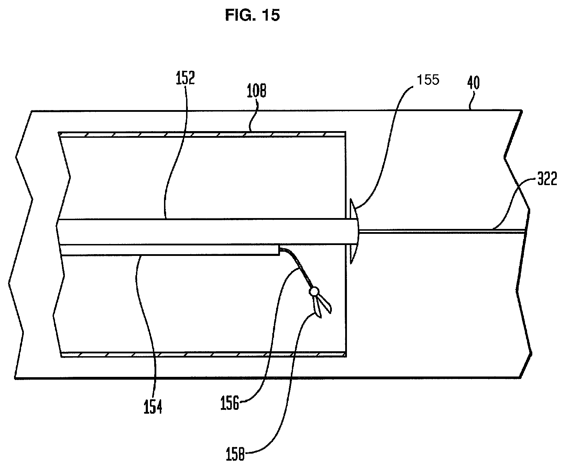

Device **100** enters and exits the patient through esophagus **302** and is ultimately positioned in its operative state, wherein pyloric columns **106** extend through pyloric sphincter **20** (e.g., see Fig. **15****).** Initially, a gastroscope **306** is lubricated, inserted into patient's mouth **307,** and fed through esophagus **302** and the gastroesophageal ("GE") junction into stomach **30,** as shown in Fig. **11****.** Gastroscope **306** is preferably approximately 9.8 millimeters in length, and preferably has approximately a 2.8 millimeter working channel and suitable viewing and recording equipment, for example. It will be understood that tools and components that are described as being passed through or inserted into gastroscope **306** are passed through or inserted into its working channel. A lubricant such as Surgilube or equivalent may be provided as needed to lubricate the bypass device and/or any of the associated surgical equipment.

Gastroscope **306** should ultimately be positioned such that its distal end **308** is adjacent to pyloric sphincter **20.** Preferably, a guidewire **322** is hydrated and inserted through gastroscope **306.** Guidewire **322** is passed through pyloric sphincter **20,** which may be aided by manipulation of gastroscope **306.** It may also be beneficial to pass a distal end **308** of gastroscope **306** through pyloric sphincter **20** in order to maneuver guidewire **322** through same. There should preferably be at least about **30-40** centimeters of the length of guidewire **322** passed distally through pyloric sphincter **20** and into small intestine **40** so that any further movement of guidewire **322** during the insertion procedure does not result in the accidental removal of the distal end of guidewire **322** to a position proximal of pyloric sphincter **20.** Of course, the length of guidewire **322** that should preferably be passed distally through pyloric sphincter **20** may vary according to different patients and/or procedures and may be less or more than **30-40** centimeters. After guidewire **322** is appropriately positioned, gastroscope **306** is removed from the patient.

Referring now to Fig. **12****,** bypass device **100** and delivery system **200** are lubricated and positioned over the guidewire **322** outside of the patient by advancing the proximal end of guidewire **322** through hole **206** in distal capsule **204** and into the inner lumen of support tube **152** (see Fig. **10A**). In some embodiments, an overtube (not shown) may be positioned over the guidewire **322** and advanced through the esophagus and into the patient's stomach. The overtube typically has an inner diameter of approximately 16 mm. However, in the preferred embodiment, an overtube is not required for implantation of bypass device **100.** A small steerable scope (not shown) may be advanced through the esophagus into stomach **30** through the pylorus and into the proximal portion of the duodenum. The scope is used to confirm the tissue of the stomach, pylorus and duodenum are robust and show not overt signs that they will not tolerate the device. In an exemplary embodiment, a small tube (not shown) may be inserted into the pylorus. The tube includes a distal inner tube-shaped balloon (not shown) that expands to a known diameter with a known volume of saline. In conjunction with the scope images and other prior imaging data, this instrument is used to determine the appropriate size of bypass device **100** to be used, particularly the appropriate size of duodenal anchor.

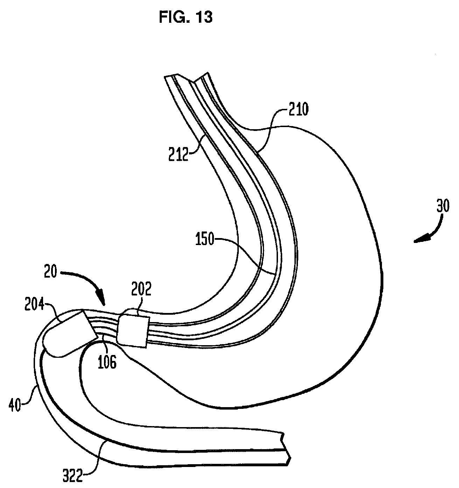

Referring now to Fig. **12****,** once the appropriate size bypass device **100** is selected, distal capsule **204** (containing sleeve **108** and duodenal anchor **104)** and proximal capsule **202** (containing gastric anchor **102** and at least a portion of columns **106)** are then advanced through the esophagus and into the stomach along guidewire **322.** Once through the esophagus, distal and proximal capsules **204, 202** separate, but remain flexibly coupled together by columns **106** and fluid tube **210** (note that tubes **210, 212** are not shown in Fig. **12****).** The capsules **202, 204** are also still aligned with each other by virtue of delivery tube structure **150** and guidewire **322** that remain extending through the entire device **100.** Once the entire device is within the stomach, support tube **152** is used to push the distal end of delivery tube structure **150** and distal capsule **204** through the pylorus into the proximal duodenum (see Fig. **13**). This may require the use of a dilator (not shown) to maintain the pylorus in its maximum diameter. Alternatively, a separate pusher rod (not shown) may be used to push the distal components of bypass device **100** into stomach **30.** It should be noted that it may not be necessary to encapsulate any components of device **100** in order to advance them through the patient's GI tract. In this case, these components will simply be advanced in their deflated configurations.

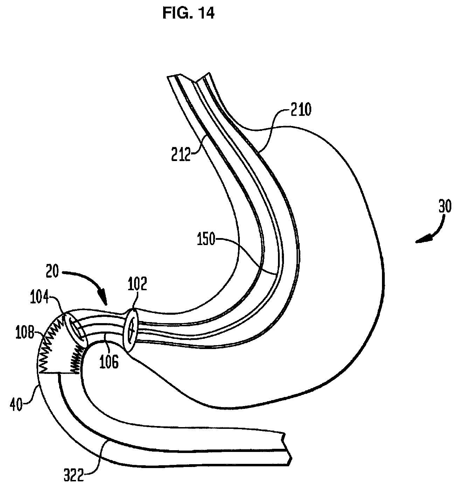

At this point, the surgeon will wait until capsules **202, 204** dissolve (unless capsules **202, 204** are not being used). It is expected that proximal capsule **202** will dissolve more rapidly, as it is designed to do so, and is subjected to a slightly more caustic environment within the stomach (alternatively, capsules **202, 204** may comprise material that can be excreted naturally by the patient). Once the proximal components of bypass device **100** are freed from capsule **202,** a fluid, such as saline, is introduced through fluid tube **212** into the interior of inflatable member **112** until it is filled to the appropriate size (see Fig. **14**). After inflating gastric anchor **102,** and after distal capsule **204** has also dissolved, fluid is delivered through fluid tube **210** into the interior of inflatable member **132** of duodenal anchor **104** in a similar fashion.

Referring now to Fig. **15****,** once duodenal anchor **104** has been inflated into its operative configuration, sleeve **108** is advanced through the duodenum and into the jejunum to its final deployment position. Specifically, support tube **152** is used to push sleeve **108** and delivery structure **150** through the patient's small intestines. Once in final position, clamp **158** is released via the actuator device or handle outside of the patient (not shown) to disengage it from the distal end of sleeve **108.**

In an alternative embodiment, the surgeon will not wait until distal capsule **204** is dissolved before advancing sleeve **108** to its final deployment position in the small intestines. In this embodiment, once capsule **204** has been advanced through the pylorus into the proximal duodenum, gastric anchor **102** is inflated as discussed above. Capsule **204** is then advanced further into the duodenum until duodenal anchor **104** is forced out of the proximal opening of capsule **204** (anchor **104** will be prevented from further distal movement by gastric anchor **102**). Capsule **204** will then be advanced through the duodenum until the distal end of sleeve **108** reaches its final deployment position. Clamp **158** is then disengaged from sleeve **108** and removed from the patient as described above and capsule **204** will eventually dissolve.

As shown in Fig. **16****,** bypass device **100** should now be in its final position with gastric anchor **102** in pyloric antrum of stomach **30** and duodenal anchor **104** just distal to the pyloric sphincter **20.** Delivery structure **150** is removed from the patient and the distal end of fluid tubes **210, 212** are cut with scissors or the like and removed. A gastroscope and/or fluoroscope (not shown) may be used to confirm the final placement of device **100.** Once device **100** is in place, guidewire **322** can be also removed from the patient.

A method for removing bypass device **100** according to the present invention will now be described. A gastroscope may be advanced through the esophagus and into the stomach **30** of patient in a suitable position for the surgeon to view the procedure. A sharp instrument (not shown) such as scissors or the like, is advanced through the patient's esophagus into stomach **30.** The sharp instrument is used to puncture gastric anchor **102** such that the fluid within the interior of membrane **112** exits into the stomach to deflate anchor **102.** Alternatively, a syringe or similar suction device (not shown) may be attached to the valve inlet **120** to withdraw the fluid from gastric anchor **102.**

Once deflated, gastric anchor is preferably positioned to the side of the antrum. A grasping or cutting instrument (not shown) is advanced through the esophagus to cut each of the pyloric columns **106** to detach gastric anchor **102** from the distal portion of device **100.** The last column **106** that is severed will be held by the grasping instrument to ensure that duodenal anchor **104** and sleeve **108** do not migrate in the distal direction after being detached from gastric anchor. At this point, a grasping tool or snare (not shown) is advanced into stomach **30** to grab gastric anchor **102** and gastric anchor **102** is then pulled through the esophagus and removed from the patient.

The sharp cutting instrument is then advanced through the pyloric sphincter **20** to puncture membrane **132** of duodenal anchor **104** to deflate duodenal anchor **104.** The grasping instrument may then be used to pull anchor **104** and sleeve **108** into stomach **30.** Once duodenal anchor **104** and sleeve **108** are within stomach **30,** they may be sliced up and removed or removed as a single unit. Subsequent to the removal of device **100,** a scope (not shown) can be used to determine if any tissue injury or insult that has been sustained by the implantation, use, or removal of device **100.** Provided no additional access to the stomach, pylorus, or duodenum is required, removal of the scope concludes the procedure.

Alternatively, the duodenal anchor **104** can be deflated without severing columns **106** and removing gastric anchor **102.** In this embodiment, the sharp instrument is advanced around gastric anchor **102,** through columns **106** and pylorus **20** to duodenal anchor **104.** Duodenal anchor is deflated and pulled into the stomach (along with sleeve **108**). The entire bypass device **100** may then be removed as a single unit, or it may be sliced up into smaller components to facilitate passage through the patient's esophagus.

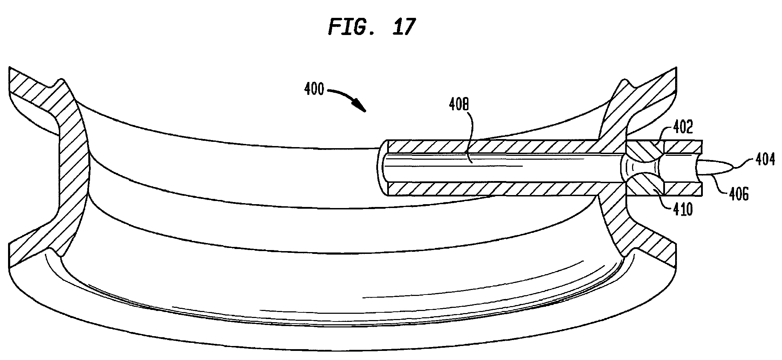

Fig. **17** illustrates one portion of an alternative embodiment of the gastric and duodenal anchors (for convenience only one of the anchors is shown in Fig. **17**). In this embodiment, the anchors both include an outer wall housing a hollow interior designed for inflation as described above. Fig. **17** illustrates a central portion **400** of the outer wall of the anchors. As shown, the anchors further include a valve **402** having an inlet **404,** a one-way valve member **406,** a fluid passage **408** and an expandable member **410** located within fluid passage **408** between the interior of the anchor and valve member **406.** Expandable member **410** preferably comprises a material designed to slowly absorb fluid upon, such as water or saline and expand with the absorbed fluid. In the preferred embodiment, expandable member **410** comprises a hydrogel material although other materials can be used that are well known in the art. To inflate the anchor, fluid is delivered through inlet **404** such that it passes via fluid passage **408** past expandable member **410** and valve member **406** into the interior of the anchor. Valve member **406** is designed to prevent the fluid from flowing back through fluid passage **408** and inlet **404.** As additional protection from this event occurring, however, expandable member **410** will absorb fluid as the fluid fills the interior of the anchor and passes back through passage **408** to saturate expandable member **410.** Expandable member **410** is designed to expand when hydrated such that it completely blocks fluid passage **408,** thereby preventing any fluid flow in either direction through fluid passage. This not only provides additional protection from fluid leakage, but also prevents any unwanted fluid (such as stomach acid and the like) from passing into the interior of the anchor.

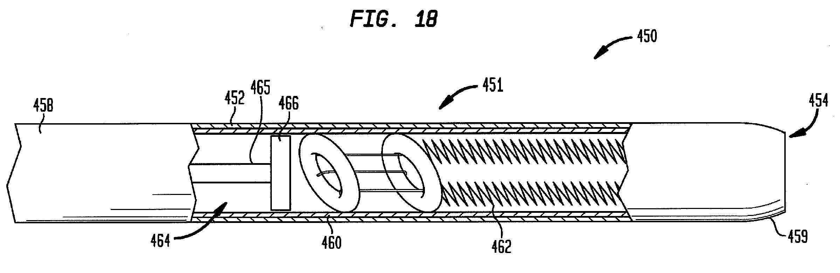

Fig. **18** illustrates an alternative embodiment of a delivery system **450** of the present invention. In this embodiment, system **450** comprises an introducer tube **451** defining an elongate shaft **452** that has sufficient flexibility to extend through a patient's esophagus into the stomach (similar to the overtube described above). Shaft **452** has an open distal end **454** and an open proximal end (not shown as the entire shaft **452** of tube **451** is not shown in Fig. **18**) and an internal lumen **458** designed as a working channel for passing instruments therethrough. Tube **451** further comprises a thin flexible bag or sleeve **460** comprising a suitable biocompatible material such as silicone or the like. Sleeve **460** houses a bypass device **462** such as one of the bypass devices described above. Sleeve **460** is preferably long enough to extend entirely through internal lumen **458** of shaft **452** to facilitate placement of sleeve **460** and device **462** in tube **451** (see below). In the preferred embodiment, sleeve **460** has open distal and proximal ends and is fastened to the proximal end of introducer tube **451** such that sleeve
**460** remains within tube **451** when device **462** is propelled from tube **451** (see below). Sleeve **460** is also sized to allow it to fit within internal lumen **458** while still housing bypass device **462** in its collapsed configuration.

System **450** may optionally include a pusher or advancing member **464** including an elongate rod **465** with a proximal handle (not shown) and a distal pusher **466** designed to press against a proximal end of bypass device **462** when bypass device **462** is loaded within lumen **458** of tube **451.** In some embodiments, handle (not shown) can be used to collapse or expand pusher **466** (discussed below). Tube **451** further comprises a tapered distal end **459** around distal opening **454** to provide for atraumatic advancement through the esophagus.

In use, bypass device **462** is placed within sleeve **460** and sleeve **460** is loaded into the distal end portion of internal lumen **458.** In a preferred embodiment, the user extends the proximal end of sleeve **460** through the entire length of lumen **458** and pulls sleeve **460** proximally such that bypass device **462** is pulled into the distal end portion of lumen **458.** Once loaded, the device **462** is ready for implantation in the patient.

To implant the device, introducer tube **451** is extended through the patient's esophagus such that its proximal opening **454** is positioned within the stomach. Bypass device **462** is then propelled out of tube **451** into the stomach. This can be accomplished by passing advancing member **464** through proximal opening of introducer tube **451** and advancing it through lumen **458** until it propels bypass device **462** out of the distal opening **454** of tube **451.** Alternatively, other devices, such as a gastroscope, can be used to propel bypass device into the stomach.

In the preferred embodiment, sleeve **460** has open proximal and distal ends and is designed to remain within lumen **458** of tube **451** as bypass device **462** is advanced into the stomach. Thus, both sleeve **462** and tube **451** can be easily removed from the patient after device **462** has been deployed. In other embodiments, sleeve **462** passes into the stomach along with device **462** and is then detached or removed from device **462.** This can be accomplished by a variety of means, such as cutting the sleeve away from the device **462.** Alternatively, sleeve **462** may already include a cut-a-way portion that can be easily detached to open sleeve **462** and allow bypass device **462** to be removed. In yet another embodiment, sleeve **462** may comprise a dissolvable material that dissolves away within the stomach (similar to the capsules discussed above). Once it has been deployed into the stomach, bypass device **462** may be passed along a guidewire and deployed into its final position as discussed above.

In certain embodiments, introducer tube **451** is long enough to extend through the esophagus and stomach and through the pylorus into the duodenum of the patient. In these embodiments, tube **451** may optionally include a bend at its distal end portion to facilitate passage of the tube **451** through the natural bend in the human stomach between the esophagus and the pylorus. Alternatively, the delivery system may include a separate overtube having such a bend. In this embodiment, the separate overtube will be first inserted through the esophagus with a separate straight introducer therein (i.e., to maintain the overtube in a substantially straight configuration as it passes through the esophagus). Once the overtube has passed through the esophagus, the straight introducer is removed such that the overtube is allowed to bend within the stomach into its natural position. At that point, the flexible introducer tube **451** housing the bypass device is advanced through the overtube to the duodenum.

In use, once the distal opening of tube **451** resides in the duodenum, the distal end portions (i.e., the sleeve and the duodenal anchor) of the bypass device are propelled out of the distal opening of the introducer tube **451** and into the duodenum of the patient. The duodenal anchor is then inflated to prevent proximal movement of the distal end portions of the bypass device. The introducer tube **451** is then retracted through the pylorus and into the stomach. As this occurs, the gastric anchor will naturally be pulled out of the distal end of the introducer tube **451** from the counterforce of the inflated duodenal anchor against the distal surface of the pylorus. The gastric anchor may then be inflated within the stomach and the sleeve extended through the duodenum as described above.

In yet another alternative embodiment, the pusher device or advancing member is designed to perform multiple functions. Namely, the pusher device is sized and shaped to fit between the gastric and duodenal anchors within the introducer tube. In this capacity, the pusher device is used to push or propel duodenal anchor and sleeve out of the distal opening of the introducer tube into the duodenum. In addition, the pusher device will function to prevent the gastric anchor from being advanced through such distal opening when the distal opening is located within the duodenum. Once the distal opening of the introducer tube has been retracted through the pylorus into the stomach, the pusher device is designed to collapse and retract proximally through the middle of the gastric anchor. It can then be expanded again to propel the gastric anchor through the distal opening of the introducer tube and into the patient's stomach.

In one embodiment, the pusher device comprises an elongate rod coupled to a umbrella-shaped or claw-shaped proximal pushing device. The rod is sized to extend through the introducer tube and through the center of the gastric anchor. The proximal pushing device is sized to reside between the gastric and duodenal anchors. Distal advancement of the rod will cause the pusher device to propel the duodenal anchor through the distal opening of the introducer tube. The pusher device further comprises an actuator at the proximal end of the rod for collapsing the umbrella-shaped pushing device such that it can be retracted proximally through the center of the gastric anchor. The actuator can then be used to expand the pushing device such that further distal advancement of the rod will cause the pusher device to propel the gastric anchor through the distal opening in the introducer tube.

In yet another embodiment, an alternative method for implanting and removing the bypass devices of the present invention is now described. In this embodiment, the hollow sleeve includes one or more projections at its distal end designed to allow an endoscopic forceps, clips, clamp or similar device to attach to the projections. The projections can be loops, strings, protuberances or the like and are preferably made of the same material as the sleeve (such as silicone). The gastric and/or duodenal anchors may also include such projections for similar purposes.

In use, the bypass device is attached to an endoscopic scope by passing an endoscopic forceps or clamping device through the working channel of the scope and attaching the forceps to the projections on the distal end of the sleeve. The physician may then attach the remainder of the bypass device to the outer surface of the scope with a shroud, sleeve or other fastening device or just simply align the bypass device with the shaft of the scope. Alternatively, the scope may be advanced through the center of the two anchors of the bypass device and the sleeve such that the entire bypass device is positioned around the scope. The scope is advanced with the distal end of the sleeve through the patient's esophagus and stomach and through the pylorus into the duodenum. The remainder of the sleeve and the anchors of the bypass device are thereby pulled into the stomach alongside the scope.

Alternatively, the physician may attach the forceps to the projections on either the gastric or duodenal anchor and advance those portions of the implant through the esophagus first (i.e., passing the device into the stomach backwards). In this embodiment, the physician may then detach the forceps from the bypass device, remove the scope and attach the forceps to the distal end of the sleeve to advance the remainder of the sleeve into the patient's stomach. To avoid retraction of the anchors back through the esophagus, the gastric anchor may be fully or partially inflated after the forceps have been detached from the anchor and before withdrawing the scope through the esophagus.

After the anchors have been advanced into the stomach of the patient, the gastric anchor is preferably either partially or fully inflated as described above to prevent movement of the gastric anchor through the pylorus into the duodenum or through the lower esophageal sphincter into the esophagus. The physician then continues to advance the scope and the distal end of the sleeve through the duodenum to a position near the final target site within the patient's intestines (either in the distal duodenum or the proximal jejunum). At this point, the physician may detach the forceps from the distal end of the sleeve and withdraw the scope and forceps back into the patient's stomach.

To prevent the sleeve from "following" the scope proximally into the stomach, the physician will preferably temporarily fixate the sleeve within the duodenum. According to the present invention, one method of temporarily fixating the sleeve is to use a detachable clip (rather than the forceps described above) to grab the projection on the distal end of the sleeve.

Once in position within the duodenum, the clip is opened and attached to mucosal tissue within the duodenum. The clip can then be deployed such that it is no longer attached to its shaft, but instead fastens the sleeve to the mucosal tissue on the inner wall of the intestines. This allows the scope and shaft of the clip device to be withdrawn while the sleeve is fixated within the duodenum.

In certain embodiments, multiple clips may be used to fasten the sleeve to the inner walls of the intestines. The clips may serve the purpose of creating an additional anchor for the device to prevent migration and to ensure that the sleeve remains patent during the period of time it is implanted within the patient. In addition, the clips will preferably be observable under fluoroscopy such that the physician can ensure that the sleeve has remained in place after implantation.

In another embodiment, the sleeve comprises one or more internal lumens extending down a portion of, or the entire length of, the sleeve. The internal lumens are preferably fluidly coupled to one of the flexible columns which are, in turn, fluidly coupled to a valve within the gastric anchor. In an exemplary embodiment, the sleeve will comprise 3 or 4 internal lumens spaced around its circumference. In use, a fluid is delivered through the gastric anchor and column and into the internal lumens of the sleeve. The fluid causes the sleeve to extend fully and inhibits proximal migration of the sleeve when the scope is retracted from the patient's duodenum. In addition, the fluid-filled lumens will ensure that the sleeve remains patent without any kinks or twists along its length.

Once the scope has been retracted from the duodenum, the duodenal anchor is advanced past the patient's pylorus into the proximal portion of the duodenum. Preferably, the scope is used to push the duodenal anchor through the pylorus. Alternatively, the forceps can be attached to one of the projections on either of the gastric or duodenal anchors to push and/or pull the duodenal anchor into position. The duodenal anchor is then inflated as described above.

In yet another alternative embodiment, the sleeve is "self-deploying" through the duodenum. In this embodiment, the sleeve is positioned within the proximal duodenum as described above and then allowed to "self-deploy" and advance through the duodenum into position. In one embodiment, the sleeve comprises internal fluid lumens as described above and the fluid is delivered into the lumens to force the sleeve to extend down the length of the duodenum. In another embodiment, the sleeve comprises a mass at its distal end (e.g., a thickened annular portion of the sleeve, one or more balls or projections attached to the distal end or the like). The mass will advance through the patient's intestines through natural peristalsis and pull the sleeve distally until it is fully extended. In yet another embodiment, the sleeve comprises a dissolvable portion at its distal end that occludes all or a portion of the distal end of the sleeve. In this embodiment, fluid is flushed through the sleeve and because its distal end is occluded causes the sleeve to extend distally. After the dissolvable portion dissolves within the intestines, the distal end of the sleeve is opened up to allow chyme to pass therethrough.

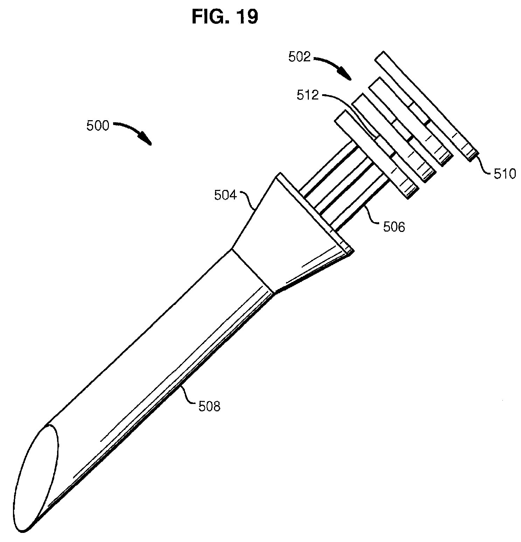

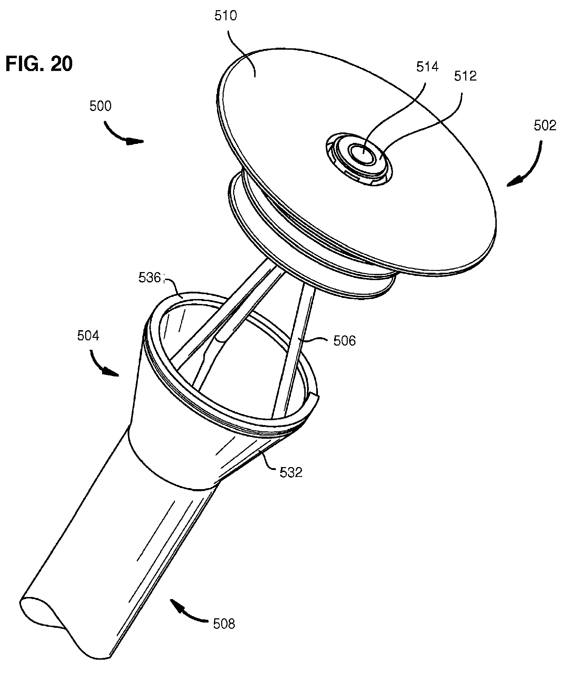

Figs. **19** and **20** illustrate an example of an obesity device **500** not according to the present invention. As shown, device **500** includes a gastric flow restrictor **502** coupled to a duodenal anchor **504** by a plurality of flexible silicone tethers or pyloric columns **506** and a hollow sleeve **508** coupled to the distal end of anchor **504.** Pyloric columns **506** are designed to extend through the pyloric sphincter **20** to allow both flow restrictor **502** and anchor **504** to move back and forth within the stomach **30** and duodenum **50,** respectively, with the natural peristalsis motion of the GI tract (see FIG. **36**). Pyloric columns **506** may be cylindrical or any other type of prismic shape and are preferably designed such that the distance between the distal end of flow restrictor **510** and the proximal end of anchor **504** is about **30** mm in the fully extended, but relaxed condition. Preferably, device **500** includes three or more pyloric columns **506,** which are attached to the distal end of flow restrictor **502,** through a button (not shown). The button is preferably comprised of a material having a greater rigidity than the rest of device **500,** and may either be co-molded into device **500** or assembled afterward as a separately manufactured component. The button is preferably configured similarly to a conventional button, being disc-shaped and having two or more bores (not shown). The button may be disposed within or adjacent to the distal-most flange. Alternatively, columns **506** may be glued to flow restrictor **502** or coupled to flow restrictor **502** during the molding process, as described above.

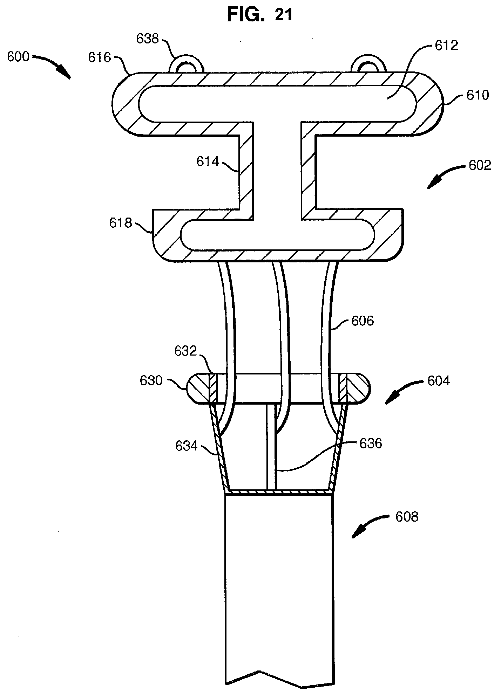

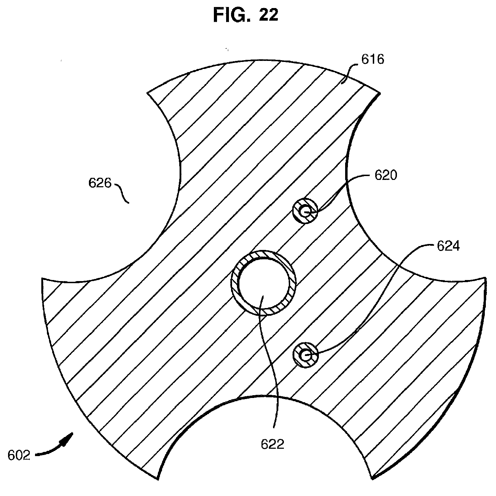

Figures **21** and **22** illustrate an alternative embodiment of an obesity device **600** that is inflated into the operative configuration. As shown, obesity device **600** comprises a flow restrictor **602** coupled to an anchor **604** with a plurality of pyloric columns **606,** and a hollow sleeve **608** coupled to anchor **604** (only the proximal portion of sleeve **608** is shown in Fig. **21**). In this embodiment, flow restrictor **602** comprises an outer collapsible shell **610** surrounding a hollow interior section **612.** Collapsible shell **610** is movable between the inflated or operative configuration shown in Fig. **21** to a deflated or collapsed configuration (not shown) for placement and removal from the patient. Collapsible shell **610** has a roughly cylindrical inner core portion **614** with proximal and distal flanges **616, 618.** Shell **610** preferably comprises a moderate durometer silicon with a wall thickness of about 0.5-4.0 mm, preferably between about 1-3 mm. Flanges **616, 618** are rounded at the circumference to provide atraumatic contact with tissue and are spaced from each other by about **10** to **40** mm, preferably between about 20 to 25 mm. Proximal flange **616** preferably has an outer diameter of about 40-60 mm and distal flange preferably has an outer diameter of about 25-35 mm. Flanges **616, 618** preferably each comprise one or more semicircular cutaways **626** to permit food passage and retrograde fluid pressure relief.

Interior section **612** is fluidly coupled to a tube **620** having an opening (not shown) at the proximal surface of proximal flange **616** for inflating interior section **612** into the operative configuration shown in Fig. **21****.** Tube **620** comprises a one-way valve (not shown), such as an umbrella valve, to ensure that fluid delivered into interior section **612** will not pass out of tube **620** after flow restrictor **602** has been inflated. Core portion **614** has an outer diameter of approximately 3-15 mm, preferably between about 4 to 6 mm, and a length of about 25-35 mm when interior section **612** is fully inflated. Flow restrictor **602** preferably includes a pair of protuberances **638** at its proximal end that can be grabbed by a simple grasping instrument to pull/manipulate the device as required during implantation/removal.

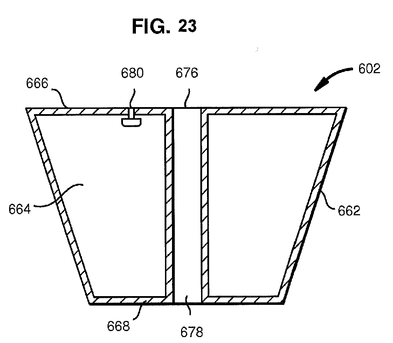

As shown in Fig. **22****,** a hollow tube **622** extends through the central axis of flow restrictor **602.** Tube **622** preferably comprises a hard plastic wall with an interior diameter of about **2-4** mm. Tube **622** provides stiffness to flow restrictor **602** as well as providing a lumen for guide wire and instrument access through flow restrictor **602** to the distal components of obesity device **600** (discussed below). In addition, a fluid lumen **624** extends through flow restrictor **602** to allow for fluid passage through one of the fluid columns **606** to inflate anchor **604** as discussed below. Fluid lumen **624** will also comprise a one-way valve (not shown) to ensure that anchor **604** remains inflated during operation of the device. Alternatively, interior **612** of flow restrictor **602** may be fluidly coupled to the interior of anchor **604** or a separate exterior fluid line (not shown) may be used to deliver fluid into anchor **604** (discussed in more detail below with respect to the embodiment shown in Figs. **23-24**).

In this embodiment, anchor **604** comprises an inflatable annular tube **630** coupled to an internal support ring **632** and a funnel **634.** Inflatable tube **630** is movable between a collapsed position for advancement through the pyloric sphincter and an expanded or inflated position wherein tube **630** has an outer diameter that is greater than the maximum diameter of the pyloric sphincter in its open position to prevent proximal migration of anchor **604** through the pyloric sphincter. Internal support ring **632** has a diameter slightly less than the maximum diameter of the pyloric sphincter and comprises a harder durometer silicone to provide stiffness and support to tube **630.** In particular, support ring **632** ensures that tube **630** maintains a substantially circular shape as it encounters intestinal forces such that the tube **630** cannot be squeezed back through pyloric sphincter by the peristalsis forces within the small intestine.

Inflatable tube **630** is preferably fluidly coupled to one or more of the bosses **636** extending from proximal to distal ends of anchor funnel **634.** Bosses **636,** in turn, are fluidly coupled to one or more of the pyloric columns **606,** i.e., through internal lumens in one or more of the bosses **636** and columns **606.** Bosses **636** preferably have some elasticity and will provide stiffness to the overall funnel shape such that anchor **604** cannot be twisted into a smaller configuration for proximal migration through the pyloric sphincter. The boss **636** that includes a fluid lumen is fluidly coupled to internal lumen **624** in flow restrictor **602.** Internal lumen **624** preferably has an inlet (not shown) at the proximal end of flow restrictor **602** allowing the physician to deliver fluid to lumen **624** and thus inflatable tube **630.** In an exemplary embodiment, the one-way valve will be positioned at the proximal end of internal lumen **624** rather than within inflatable tube **610** as discussed above. In this embodiment, the one-way valve will preferably have a mechanism that will allow for opening of the value and removal of fluid from device **600** during removal of device **600** from the patient (discussed in detail below).

In addition to, or as an alternative to, the one-way valve, a curable fluid, such as silicone, may be injected into the fluid pathway after the saline. The curable fluid will cure and harden, thereby preventing any fluid egress from the interior of tube **630.** In another embodiment, the inflatable members of the flow restrictor and/or the anchor may comprise a material that self- seals when punctured with a very sharp small instrument such as a syringe. In this embodiment, the inflatable members of the obesity device may simply be inflated with a syringe and then self-sealed by the material to prevent fluid egress.

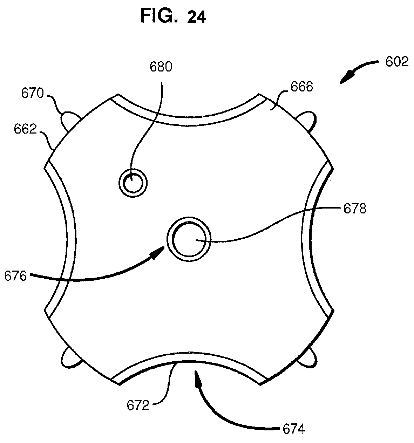

Referring now to Figs. **23** and **24****,** another preferred embodiment of a flow restrictor **660** is illustrated. Similar to the previous embodiment, flow restrictor **660** comprises an inflatable outer membrane **662** surrounding a hollow interior **664** such that membrane **662** can be inserted into the patient's stomach in a deflated or collapsed configuration and then inflated into the operative configuration. In this embodiment, membrane **662** comprises a relative thin flexible material, such as silicone, preferably having a wall diameter of about 0.1 to 1 mm thick. Flow restrictor **660** further comprises proximal and distal sheets **666, 668** that may be molded with, or glued to, membrane **662** as discussed. Alternatively, sheets **666, 668** and membrane **662** may be constructed as a single piece such that membrane **662** constitutes the entire outer wall of the structure. Proximal and distal sheets **666, 668** preferably have a slightly greater thickness (e.g., preferably between about 0.5 mm to 2.0 mm) and stiffness than membrane **662** to provide flow restrictor **660** with support and to hold its annular shape in the operative configuration.

Flow restrictor **660** further comprises one or more ribs or bosses **670** and one or more support members **672** extending along membrane **662** from proximal sheet **666** to distal sheet **668.** Bosses **670** and support members **672** preferably comprise a slightly thicker silicone material (i.e., preferably about **1.0** mm to **2.0** mm thick) and provide further support to the overall structure of flow restrictor **660.** In an exemplary embodiment, support members **672** have an arcuate shape and form cut-a-ways or recesses **674** in the otherwise conical shape of flow restrictor **660.** Recesses **674** provide paths for passage of chyme in stomach **30** past flow restrictor **660** to the pyloric sphincter and for passage of gases and/or fluids between the stomach and the duodenum. In the exemplary embodiment, bosses **670** are formed on the outside surface of membrane **662** and support members **672** are formed within the inner surface of membrane **662.** However, it will be recognized by those skilled in the art that a variety of different configurations can be employed to provide structure to flow restrictor **660** in the inflated configuration.

Flow restrictor **660** further includes a central tube **676** having an inner lumen **678** extending along its longitudinal axis and a one-way valve **680** with an opening in distal sheet **666.** Central tube **676** preferably has a wall thickness of about 0.1 mm to 1 mm and an inner diameter of between about 2 mm to 4 mm. Central tube **676** provides additional structural support for flow restrictor **660** and also provides a lumen for passage of a guidewire and/or other instruments, such as a gastroscope. One-way valve **680** is configured for coupling to a fluid tube (not shown) for delivering a fluid, such as saline, to the interior **664** of membrane **662** for inflation of flow restrictor **660.**

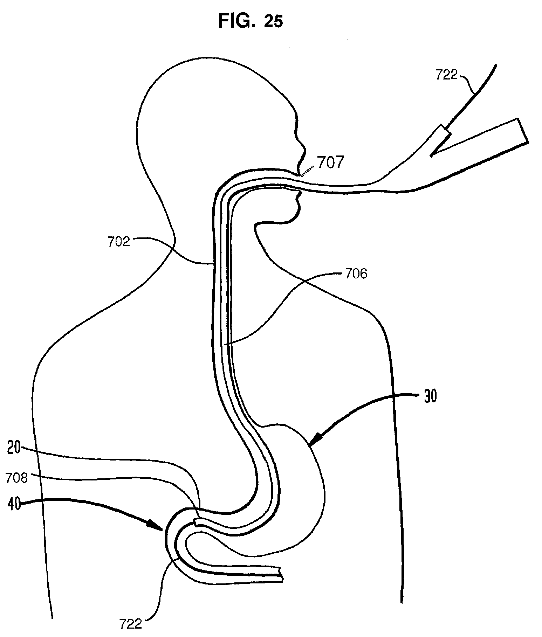

In reference to Figs. **25-31****,** a method of implanting and removing an obesity device **800** (similar to one of, or a combination of, the embodiments shown in Figs. **19** - **24** will now be described. While the description of this method will be specifically directed to the embodiments illustrated above, it will be understood by those skilled in the art that this method (or similar methods) can be used to implant and remove all of the embodiments of the present invention, including embodiments or designs that may not be specifically described or illustrated herein.

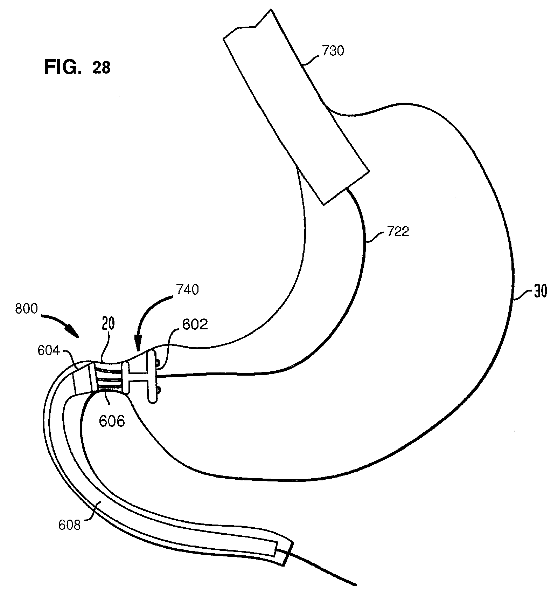

Device **800** enters and exits the patient through esophagus **702** and is ultimately positioned in its operative state, wherein pyloric columns **606** (shown in Fig. **21**) extend through pyloric sphincter **20** (e.g., see Fig. **28**). In certain embodiments, flow restrictor **602** (Fig. **21**) and anchor **604** (Fig. **21****)** are separately encapsulated for implantation such that each may be independently released. Ideally, anchor 604 is released first such that anchor 604 and sleeve **608** (Fig. **21**) are advanced as a single uninflated structure into the duodenum, coupled to flow restrictor **602** by columns **606.** The flow restrictor **602** is then inflated following the proper positioning of anchor 604 and sleeve **608.** In other embodiments, the flow restrictor and anchor are not encapsulated and are simply advanced through the esophagus or the overtube in their deflated configurations.

Initially, a gastroscope **706** is lubricated, inserted into patient's mouth **707,** and fed through esophagus **702** and the gastroesophageal ("GE") junction into stomach **20,** as shown in Fig. **25****.** Gastroscope **706** is preferably approximately **9.8** millimeters in length, and preferably has approximately a **2.8** millimeter working channel and suitable viewing and recording equipment, for example. It will be understood that tools and components that are described as being passed through or inserted into gastroscope **706** are passed through or inserted into its working channel. A lubricant such as Surgilube or equivalent may be provided as needed to lubricate the obesity device and/or any of the associated surgical equipment.

Gastroscope **706** should ultimately be positioned such that its distal end **708** is adjacent to pyloric sphincter **20.** Preferably, a guidewire **722** is hydrated and inserted through gastroscope **706.** Guidewire **722** is passed through pyloric sphincter **20,** which may be aided by manipulation of gastroscope **706.** It may also be beneficial to pass a distal end **708** of gastroscope **706** through pyloric sphincter **20** in order to maneuver guidewire **722** through same. There should preferably be at least about **30-40** centimeters of the length of guidewire **722** passed distally through pyloric sphincter **20** and into small intestine **40** so that any further movement of guidewire **722** during the insertion procedure does not result in the accidental removal of the distal end of guidewire **722** to a position proximal of pyloric sphincter **20.** Of course, the length of guidewire **722** that should preferably be passed distally through pyloric sphincter **20** may vary according to different patients and/or procedures and may be less or more than **30-40** centimeters. After guidewire **722** is appropriately positioned, gastroscope **706** is removed from the patient.

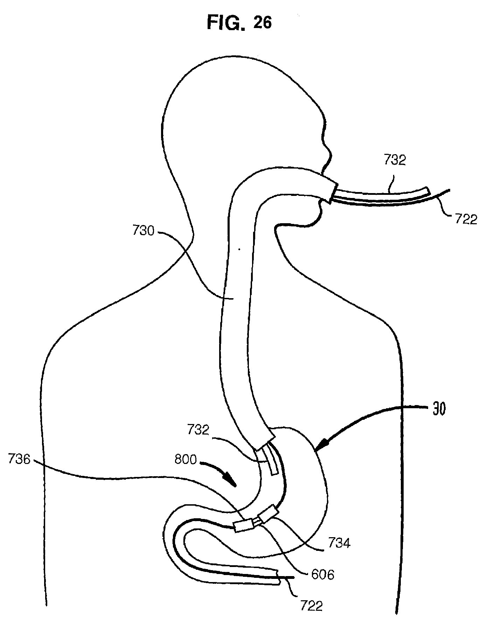

Referring now to Fig. **26****,** an overtube **730** is positioned over the guidewire **722** and advanced through the esophagus and into the patient's stomach. Overtube **730** typically has an inner diameter of approximately **16** mm. Obesity device **800** is lubricated and positioned over the guidewire **722** outside of the patient. Once overtube **730** is positioned within stomach **30,** a small steerable scope (not shown) is advanced through overtube **730** into stomach **30** through the pylorus and into the proximal portion of the duodenum. The scope is used to confirm the tissue of the stomach, pylorus and duodenum are robust and show not overt signs that they will not tolerate the device. In an exemplary embodiment, a small tube (not shown) may be inserted into the pylorus. The tube includes a distal inner tube-shaped balloon (not shown) that expands to a known diameter with a known volume of saline. In conjunction with the scope images and other prior imaging data, this instrument is used to determine the appropriate size of obesity device **800** to be used, particularly the appropriate size of anchor 604.

Once the appropriate size obesity device **800** is selected, a pusher rod **732** is used to push obesity device **800** into stomach **30.** In the exemplary embodiment, flow restrictor **660** is encapsulated within a proximal capsule **734** and both anchor 604 and sleeve **608** are preferably encapsulated within a distal capsule **736** to help advance device **800** through overtube **730.** It should be noted that it may not be necessary to encapsulate flow restrictor **660** and anchor **604** in order to advance them through overtube **730.** In this case, these components will simply be advanced through overtube **730** in their deflated configurations.

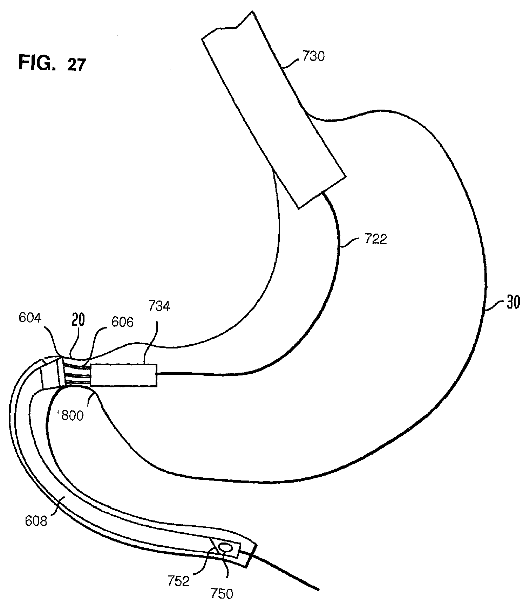

Referring now to Fig. **27****,** distal capsule **736** is removed and anchor **630** and sleeve **608** are advanced with pusher rod **732** (not shown in Fig. **27**) through the pylorus and into the proximal portion of the duodenum. This may require the use of a dilator (not shown) to maintain the pylorus in its maximum diameter. Once positioned properly, a fluid, such as saline or another appropriate fluid is injected through an external tube (not shown) and one-way valve into the expandable membrane to inflate the membrane to its expanded or operative configuration. The external tube would then be cut by a pair of scissors or other such instrument that can be advanced through the working channel of overtube **730.** Alternatively, the fluid may be delivered through a syringe directly into the membrane or through an internal tube within the flow restrictor that is coupled to the interior of the membrane as described previously.

Once the anchor **630** has been inflated into its operative configuration, knob **750** at the distal end of sleeve **608** is grasped by a suitable grasping instrument and advanced downwardly through the duodenum and into the jejunum. When sleeve **608** has reached its maximum length, perforation **752** is torn and sleeve **608** is fully deployed. Knob **750** and perforation **752** can then be removed from the patient. Alternatively, sleeve **608** may be positioned either before anchor **630** has been inflated or after flow restrictor **660** has been inflated as described below.

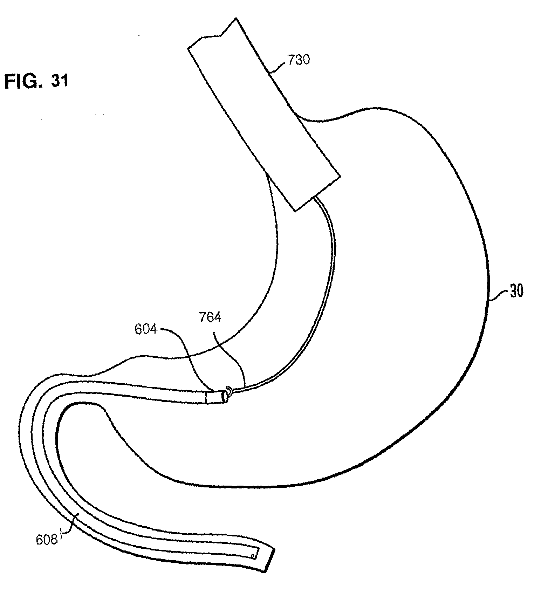

Referring now to Fig. **28****,** proximal capsule **734** (shown in Fig. **27**) is then disengaged from flow restrictor **660** and removed from the patient. A fluid, such as saline, is injected through inlet **680** (see Fig. **31****)** into the interior portion **664** of flow restrictor **660** to inflate flow restrictor **660** into its operative configuration. Obesity device **800** should now be in its final position with flow restrictor **660** in pyloric antrum **740** of stomach **30** and expandable membrane **642** of anchor **630** just distal to the pyloric sphincter **20.** A gastroscope and/or fluoroscope (not shown) may be used to confirm the final placement of device **800.** Once device **800** is in place, guidewire **722** and overtube **730** can be removed from the patient.

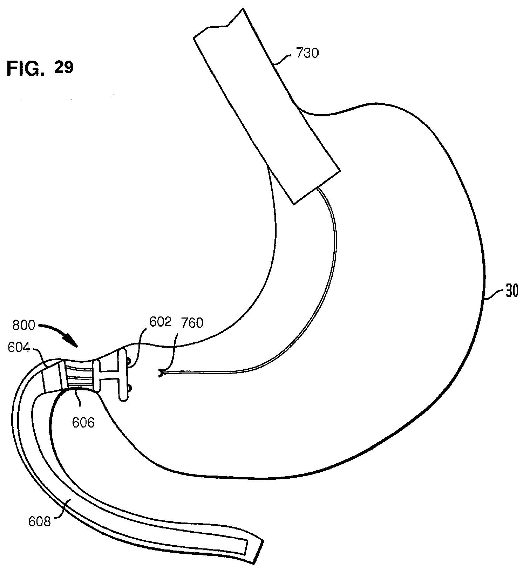

Referring now to Figs. **29-31****,** a method for removing obesity device **800** will now be described. As shown in Fig. **21****,** overtube **730** is advanced through the esophagus and into position within the stomach **30** of patient and a gastroscope (not shown) is deployed through overtube **730** in a suitable position for the surgeon to view the procedure. A sharp instrument **760,** such as scissors or the like, is advanced through overtube **730** into stomach **30.** Sharp instrument **760** is used to puncture flow restrictor **660** such that the fluid within interior portion **664** of flow restrictor **660** exits interior portion **664** into the stomach to deflate flow restrictor **660.** Alternatively, a syringe or similar suction device (not shown) may be attached to inlet **680** to withdraw the fluid from flow restrictor **660.**

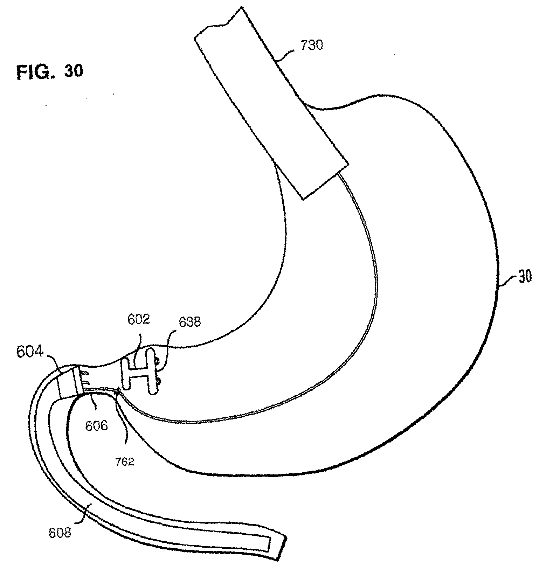

Referring now to Fig. **30****,** flow restrictor **660** is preferably positioned to the side of antrum **740.** A grasping or cutting instrument **762** is advanced through overtube **730** to cut each of the pyloric columns **606** to detach flow restrictor **660** from the distal portion of device **800.** The last column **606** that is severed will be held by grasping instrument **762** to ensure that anchor **630** and sleeve **608** do not migrate in the distal direction after being detached from flow restrictor **660.** At this point, a grasping tool or snare (not shown) is advanced into stomach **30** to grab one or both of protuberances **638** on the proximal surface of flow restrictor **660.** Flow restrictor **660** is then pulled through overtube **730** and removed from the patient.

Referring now to Fig. **31****,** a grasping instrument **764** is then advanced through the pyloric sphincter **20** to grasp a ball element attached to wire embedded in the inner membrane of the ring-inner tube structure. Grasping instrument **764** is pulled to cause the fluid within membrane **642** to exit membrane **642** and deflate anchor **630.** Grasping instrument **764** may then be used to pull anchor **630** and sleeve **608** into stomach **30.** In alternative embodiments, membrane **642** is fluidly coupled to a lumen (not shown) within flow restrictor **660** through one or more of the boss(es) **635** and column(s) **606.** In these embodiments, cutting of the column(s) **606** will automatically deflate membrane **642.**

Once anchor **630** and sleeve **608** are within stomach **30,** they may be sliced up and removed or removed as a single unit. Subsequent to the removal of obesity device **800,** a scope (not shown) can be used to determine if any tissue injury or insult that has been sustained by the implantation, use, or removal of device **800.** Provided no additional access to the stomach, pylorus, or duodenum is required, removal of the scope and overtube **730** conclude the procedure.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the scope of the present invention as defined by the appended claims.

## Claims

1. A device (100) for treating a patient with obesity and/or type II diabetes comprising:
- a gastric anchor (102) movable between a first configuration sized and shaped for advancement through an esophagus into a selected region of a stomach of the patient and a second configuration sized and shaped for inhibiting distal movement of the gastric anchor through a pyloric sphincter (20) of the patient;
- a duodenal anchor (104, 230) coupled to the gastric anchor and movable between a first configuration sized and shaped for advancement through the esophagus, the stomach and the pyloric sphincter (20) into a proximal region of a duodenum of the patient and a second configuration sized and shaped to inhibit proximal movement of the duodenal anchor through the pyloric sphincter;
- a substantially hollow sleeve (108) coupled to the duodenal anchor and sized and shaped to extend through at least a proximal portion of the duodenum of the patient; and
- at least one flexible column (106) coupling the gastric anchor to the duodenal anchor and configured for positioning across the pyloric sphincter of the patient,
**characterized by**:
- the gastric anchor being configured for inflation by a fluid from a collapsed configuration to an inflated configuration;
- the duodenal anchor being configured for inflation by a fluid from a collapsed configuration to an inflated configuration, the gastric anchor comprising a ring (240), the ring comprising sufficiently rigid material to withstand natural peristaltic forces of the patient and remain in the second configuration, the ring being coupled to an inflatable flexible membrane (242), wherein a wire is incorporated into a seal of the membrane (242) to the ring and wherein a ball is coupled to the wire and located external to the ring and the membrane, the ball being configured, upon being grasped by an endoscopic device and being pulled, to cause the wire to tear through the membrane and the seal, thus rupturing the membrane and deflating the gastric anchor; and
- the flexible columns allowing both anchors to move back and forth within the stomach and the duodenum, respectively, with the natural peristalsis motion of the patient.

2. The device of claim 1 wherein the gastric anchor defines an exterior wall housing an interior portion and an inlet in the exterior wall for delivering a fluid to the interior portion.

3. The device of claim 1 wherein the gastric anchor has a substantially annular shape in the second configuration with an outer diameter of between about 30 mm to about 70 mm.

4. The device of claim 1 wherein the duodenal anchor defines an exterior wall housing an interior portion and an inlet in the exterior wall for delivering the fluid to the interior portion.

5. The device of claim 1 wherein the duodenal anchor has a substantially annular shape in the inflated configuration with an outer diameter of between about 20 mm to about 40 mm.

6. The device of claim 1 wherein the hollow sleeve has a length of about 1 to 3 feet of the patient.

7. The device of claim 1 wherein the gastric anchor is sized and shaped in the second configuration to apply at least periodic contact pressure to a distal portion of a pyloric antrum of the stomach, the contact pressure being sufficient to modulate parasympathetic nerves within the distal portion of the pyloric sphincter.

8. The device of claim 1 wherein the duodenal anchor is sized and shaped in the second configuration to apply at least periodic contact pressure to a proximal portion of the duodenum, the contact pressure being sufficient to modulate parasympathetic nerves within the proximal portion of the pyloric sphincter.

## Patentansprüche

1. Vorrichtung (100) zum Behandeln eines Patienten mit Fettsucht oder Diabetes Typ II, umfassend:
- eine Gastral-Verankerung (102), die zwischen einer ersten Konfiguration, die zur Beförderung durch eine Speiseröhre in eine ausgewählte Region eines Magens des Patienten bemessen und geformt ist, und einer zweiten Konfiguration, die zum Hemmen von distaler Bewegung der Gastral-Verankerung durch einen Pylorusschließmuskel (20) des Patienten bemessen und geformt ist, beweglich ist;
- eine Duodenal-Verankerung (104, 230), die an die Gastral-Verankerung gekoppelt und zwischen einer ersten Konfiguration, die zur Beförderung durch die Speiseröhre, den Magen und den Pylorusschließmuskel (20) in eine proximale Region eines Duodenums des Patienten bemessen und geformt ist, und einer zweiten Konfiguration, die zum Hemmen von proximaler Bewegung der Duodenal-Verankerung durch einen Pylorusschließmuskel bemessen und geformt ist, beweglich ist;
- eine im Wesentlichen hohle Hülse (108), die an die Duodenal-Verankerung gekoppelt und zum Erstrecken durch zumindest einen proximalen Abschnitt des Duodenums des Patienten bemessen und geformt ist; und
mindestens eine flexible Säule (106) zum Koppeln der Gastral-Verankerung an die Duodenal-Verankerung und zum Positionieren über den Pylorusschließmuskel des Patienten konfiguriert,
**dadurch gekennzeichnet, dass**:
- die Gastral-Verankerung zum Aufblasen durch ein Fluid von einer eingefallenen Konfiguration zu einer aufgeblasenen Konfiguration konfiguriert ist;
- die Duodenal-Verankerung zum Aufblasen durch ein Fluid von einer eingefallenen Konfiguration zu einer aufgeblasenen Konfiguration konfiguriert ist, wobei die Gastral-Verankerung einen Ring (240) umfasst, wobei der Ring ein ausreichend starres Material umfasst, um natürlichen peristaltischen Kräften des Patienten zu widerstehen und in der zweiten Konfiguration zu verbleiben, wobei der Ring an eine aufblasbare flexible Membran (242) gekoppelt ist, wobei ein Draht in eine Versiegelung der Membran (242) zu dem Ring eingebracht ist und wobei ein Ball an den Draht gekoppelt und extern am Ring und der Membran lokalisiert ist, wobei der Ball, nachdem er von einer endoskopischen Vorrichtung gegriffen und gezogen wurde, derart konfiguriert ist, dass er bewirkt, dass der Draht durch die Membran und die Versiegelung gezogen wird, wodurch die Membran zerrissen wird und die Gastral-Verankerung zusammenfällt; und
- die flexiblen Säulen es ermöglichen, dass beide Verankerungen im Magen und Duodendum mit der natürlichen peristaltischen Bewegung des Patienten jeweils zurück und vor bewegt werden.

2. Vorrichtung nach Anspruch 1, wobei die Gastral-Verankerung eine einen Innenabschnitt beherbergende Außenwand und einen Einlass in der Außenwand zum Abgeben eines Fluids an den Innenabschnitt definiert.

3. Vorrichtung nach Anspruch 1, wobei die Gastral-Verankerung in der zweiten Konfiguration eine im Wesentlichen ringförmige Gestalt mit einem Außendurchmesser zwischen etwa 30 mm und etwa 70 mm aufweist.

4. Vorrichtung nach Anspruch 1, wobei die Duodenal-Verankerung eine einen Innenabschnitt beherbergende Außenwand und einen Einlass in der Außenwand zum Abgeben eines Fluids an den Innenabschnitt definiert.

5. Vorrichtung nach Anspruch 1, wobei die Duodenal-Verankerung in der aufgeblasenen Konfiguration eine im Wesentlichen ringförmige Gestalt mit einem Außendurchmesser zwischen etwa 20 mm und etwa 40 mm aufweist.

6. Vorrichtung nach Anspruch 1, wobei die hohle Hülse eine Länge von etwa 1 bis 3 Fuß des Patienten aufweist.

7. Vorrichtung nach Anspruch 1, wobei die Gastral-Verankerung in der zweiten Konfiguration zum Anlegen zumindest von periodischem Kontaktdruck an einen distalen Abschnitt eines Pylorusantrums des Magens bemessen und geformt ist, wobei der Kontaktdruck zum Modulieren von parasympathetischen Nerven im distalen Abschnitt des Pylorusschließmuskels ausreichend ist.

8. Vorrichtung nach Anspruch 1, wobei die Duodenal-Verankerung in der zweiten Konfiguration zum Anlegen zumindest von periodischem Kontaktdruck an einen proximalen Abschnitt des Duodenums bemessen und geformt ist, wobei der Kontaktdruck zum Modulieren von parasympathetischen Nerven im proximalen Abschnitt des Pylorusschließmuskels ausreichend ist.

## Revendications

1. Dispositif (100) pour traiter un patient atteint d'obésité et/ou de diabète de type II, comprenant :
- une ancre gastrique (102) mobile entre une première configuration dimensionnée et façonnée pour progresser à travers l'oesophage jusque dans une région sélectionnée de l'estomac du patient et une deuxième configuration dimensionnée et façonnée pour inhiber un mouvement distal de l'ancre gastrique à travers le sphincter pylorique (20) du patient ;
- une ancre duodénale (104, 230) couplée à l'ancre gastrique et mobile entre une première configuration dimensionnée et façonnée pour progresser à travers l'oesophage, l'estomac et le sphincter pylorique (20) jusque dans une région proximale du duodénum du patient et une deuxième configuration dimensionnée et façonnée pour inhiber un mouvement proximal de l'ancre duodénale à travers le sphincter pylorique ;
- un manchon sensiblement creux (108) couplé à l'ancre duodénale et dimensionné et façonné pour s'étendre à travers au moins une partie proximale du duodénum du patient ; et
- au moins une colonne flexible (106) couplant l'ancre gastrique à l'ancre duodénale et configurée pour un positionnement à travers le sphincter pylorique du patient,
**caractérisé en ce que** :
- l'ancre gastrique est configurée pour un gonflage par un fluide à partir d'une configuration affaissée à une configuration gonflée ;
- l'ancre duodénale est configurée pour un gonflage par un fluide à partir d'une configuration affaissée à une configuration gonflée, l'ancre gastrique comprenant un anneau (240), l'anneau comprenant un matériau suffisamment rigide pour résister à des forces péristaltiques naturelles du patient et rester dans la deuxième configuration, l'anneau étant couplé à une membrane souple gonflable (242), dans lequel un fil est incorporé dans un joint d'étanchéité de la membrane (242) jusqu'à l'anneau et dans lequel une bille est couplée au fil et située à l'extérieur de l'anneau et de la membrane, la bille étant configurée de sorte que, après avoir été saisie par un dispositif endoscopique et tirée, le fil transperce la membrane et le joint d'étanchéité, provoquant ainsi une rupture de la membrane et un dégonflage de l'ancre gastrique ; et
- les colonnes flexibles permettent aux deux ancres de se déplacer d'avant en arrière au sein de l'estomac et du duodénum, respectivement, avec le mouvement péristaltique naturel du patient.

2. Dispositif selon la revendication 1, dans lequel l'ancre gastrique définit une paroi extérieure abritant une partie intérieure et une entrée dans la paroi extérieure pour délivrer un fluide à la partie intérieure.

3. Dispositif selon la revendication 1, dans lequel l'ancre gastrique a une forme sensiblement annulaire dans la seconde configuration avec un diamètre extérieur compris entre environ 30 mm et environ 70 mm.

4. Dispositif selon la revendication 1, dans lequel l'ancre duodénale définit une paroi extérieure abritant une partie intérieure et une entrée dans la paroi extérieure pour délivrer un fluide à la partie intérieure.

5. Dispositif selon la revendication 1, dans lequel l'ancre duodénale a une forme sensiblement annulaire dans la configuration gonflée avec un diamètre extérieur compris entre environ 20 mm et environ 40 mm.

6. Dispositif selon la revendication 1, dans lequel le manchon creux a une longueur d'environ 1 à 3 pieds du patient.

7. Dispositif selon la revendication 1, dans lequel l'ancre gastrique est dimensionnée et façonnée dans la seconde configuration pour appliquer au moins une pression de contact périodique à une partie distale d'un antre pylorique de l'estomac, la pression de contact étant suffisante pour moduler des nerfs parasympathiques au sein de la partie distale du sphincter pylorique.

8. Dispositif selon la revendication 1, dans lequel l'ancre duodénale est dimensionnée et façonnée dans la seconde configuration pour appliquer au moins une pression de contact périodique à une partie proximale du duodénum, la pression de contact étant suffisante pour moduler des nerfs parasympathiques au sein de la partie proximale du sphincter pylorique.
